# EUROPEAN PATENT SPECIFICATION

(11) **EP 2 249 825 B1**
(45) Date of publication and mention of the grant of the patent: **07.10.2015**
(21) Application number: 08726839.7
(22) Date of filing: 14.03.2008
(51) Int. Cl.: A61K 31/255, A61K 31/095, A61P 39/00, A61P 39/06

(54) **TREATMENT METHODS AND COMPOSITIONS FOR LUNG CANCER, ADENOCARCINOMA, AND OTHER MEDICAL CONDITIONS**
BEHANDLUNGSVERFAHREN UND ZUSAMMENSETZUNGEN GEGEN LUNGENKREBS, ADENOKARZINOM UND ANDERE MEDIZINISCHE KRANKHEITSZUSTÄNDE
PROCEDES DE TRAITEMENT POUR LE CANCER PULMONAIRE, L'ADENOCARCINOME, ET D'AUTRES ETATS PATHOLOGIQUES

(43) Date of publication of application: 17.11.2010
(73) Proprietor: BIONUMERIK PHARMACEUTICALS, INC., San Antonio, TX 78230 (US)
(72) Inventor: HAUSHEER, Frederick, H., Boerne TX 78015 (US)
(74) Representative: Lucas, Brian Ronald
(86) International application number: PCT/US2008/003405
(87) International publication number: WO 2009/113983

(56) References cited:
- WO-A2-2007/109184
- US-A- 5 902 610
- US-A- 6 037 336
- US-A- 6 040 312
- US-A1- 2005 256 055
- LERZA R ET AL: "STUDIES ON HEMOTOXICITY OF CYCLOPHOSPHAMIDE DOXORUBICIN AND CIS DIAMMINEDICHLOROPLATINUM COMBINED WITH SODIUM 2 MERCAPTOETHANESULFONATE", TUMORI, vol. 74, no. 3, 1988, pages 333-338, XP009151536, ISSN: 0300-8916
- NISHIMORI T ET AL: "GENERAL PHARMACOLOGICAL EFFECTS OF MESNA AND ITS METABOLITE DIMESNA", OYO YAKURI - PHARMACOMETRICS, OYO YAKURI KENKYUKAI, JP, vol. 39, no. 5, 1 January 1990 (1990-01-01), pages 529-549, XP009151501, ISSN: 0300-8533
- HOWS J M ET AL: "Comparison of mesna with forced diuresis to prevent cyclophosphamide induced haemorrhagic cystitis in marrow transplantation: A prospective randomised study", BRITISH JOURNAL OF CANCER 1984 GB, vol. 50, no. 6, 1984, pages 753-756, XP000002657570, ISSN: 0007-0920
- M VERSCHRAAGEN ET AL: "Possible (enzymatic) routes and biological sites for metabolic reduction of BNP7787, a new protector against cisplatin-induced side-effects", BIOCHEMICAL PHARMACOLOGY, vol. 68, no. 3, 1 August 2004 (2004-08-01) , pages 493-502, XP55005454, ISSN: 0006-2952, DOI: 10.1016/j.bcp.2004.04.005
- CHU ET AL.: 'Taxanes as first-line therapy for advanced non-small cell lung cancer: a systematic eview and practice guideline' LUNG CANCER vol. 50, no. 3, December 2005, pages 355 - 374, XP025286893
- GRECO ET AL.: 'Gemcitabine. Carboplatin, and Paclitaxel for Patients With Carcinoma of Unknown Primary Site: A Minnie Pearl Cancer Research Network Study' J CLIN ONCOL vol. 20, no. 6, 15 March 2002, pages 1651 - 1656, XP008140012

## Description

### FIELD OF THE INVENTION

The present invention relates to compounds for use in mitigating or preventing chemotherapy-induced anemia in patients suffering from lung cancer or adenocarcinoma.

### BACKGROUND OF THE INVENTION

Currently, there is a substantial, unmet need for medicaments that can improve the survival of patients with cancer and/or slow the progression of their tumor(s). There is also a need for medicaments that can stimulate or maintain the beneficial physiological function of important bodily processes in normal *(i.e.,* non-cancerous) cells and tissues.

In brief, there are disclosed herein: (i) compositions, methods, and kits which lead to an increase in patient survival time in cancer patients receiving chemotherapy; (ii) compositions and methods which cause cytotoxic or apoptotic potentiation of the anti-cancer activity of chemotherapeutic agents; (iii) compositions and methods for maintaining or stimulating hematological function in patients in need thereof, including those patients suffering from cancer; (iv) compositions and methods for maintaining or stimulating erythropoietin function or synthesis in patients in need thereof, including those patients suffering from cancer; (v) compositions anu methods for mitigating or preventing anemia in patients in need thereof, including those patients suffering from cancer; (vi) compositions and methods for maintaining or stimulating pluripotent, multipotent, and unipotent normal stem cell function or synthesis in patients in need thereof, including those patients suffering from cancer; (vii) compositions and methods which promote the arrest or retardation of tumor progression in cancer patients receiving chemotherapy; (viii) compositions and methods for increasing patient survival and/or delaying tumor progression while maintaining or improving the quality of life in a cancer patient receiving chemotherapy; (ix) novel methods of the administration of taxane and/or platinum medicaments and a Formula (I) compound of the present invention to a cancer patient; and (x) kits to achieve one or more of the aforementioned physiological effects in a patient in need thereof, including those patients suffering from cancer.

The compositions used in the present invention comprise a therapeutically effective amount of a Formula (I) compound, specifically 2,2'-dithio-bis-ethane sulfonate or a pharmaceutically-acceptable salt thereof. Compositions of Formula (I), and their synthesis, are described in published U.S. Patent Application No. 2005/0256055. It should be noted that *all* of the aforementioned chemical entities in the previous three (3) sentences are included in the terms "Formula (I) compounds" and "Formula (I) compositions" as utilized herein, unless otherwise specifically stated, including the disodium salt of 2,2'-dithio-bis-ethane sulfonate (referred to in the literature as dimesna, Tavocept™, and BNP7787) and the metabolite of disodium 2,2'-dithio-bis-ethane sulfonate, known as 2-mercapto ethane sulfonate sodium (referred to in the literature as mesna).

Recently, surprising and medically-important new finding and functions, based upon recent clinical trial results, have been observed involving the Formula (I) compounds. These observation have extremely important implications for the treatment of cancer and other medical conditions.

### I. Lung Cancer

Lung cancer is the leading cause of smoking- and cancer-related mortality in both sexes. The prevalence of lung cancer is second only to that of prostate cancer in men and breast cancer in women. Lung cancer recently surpassed heart disease as the leading cause of smoking-related mortality. Most lung carcinomas are diagnosed at an advanced stage, conferring a poor prognosis. Lung cancer is estimated to be the cause of 921,000 deaths each year worldwide, accounting for approximately 18% of all cancer-related deaths. Lung cancer is highly lethal, with a 5-year patient survival rate of only 14% being observed in the United States. An estimated 164,100 (*i.e.,* 89,500 in men and 74,600 in women) new lung cancer cases will occur this year (2008) in the United States. *See, e.g.,* National Cancer Institute-2008 Lung Cancer Estimates (www.Cancer.gov).

Lung cancer manifests with symptoms produced by the primary tumor, locoregional spread, metastatic disease, or ectopic hormone production. Approximately 7-10% of patients with lung cancer are asymptomatic and their cancers are diagnosed incidentally after a chest x-ray performed for other reasons. The symptoms produced by the primary tumor depend on its location (*e.g*., central, peripheral).

Of the symptoms produced by the primary tumor, central tumors are generally squamous cell carcinomas and produce symptoms of cough, dyspnea, atelectasis, post-obstructive pneumonia, wheezing, and hemoptysis and peripheral tumors are generally adenocarcinomas or large cell carcinomas and, in addition to causing cough and dyspnea, can cause symptoms due to pleural effusion and severe pain as a result of infiltration of parietal pleura and the chest wall. Symptoms due to locoregional spread can include: (i) superior vena cava obstruction; (ii) paralysis of the left recurrent laryngeal nerve and phrenic nerve palsy (causing hoarseness and paralysis of the diaphragm); (iii) pressure on the cervical sympathetic plexus (causing Horner syndrome); (iv) dysphagia resulting from esophageal compression; (v) pericardial effusion and cardiac tamponade; and (vi) superior sulcus apical primary tumors can cause compression of the brachial plexus roots as they exit the neural foramina, causing intense, radiating neuropathic pain in the ipsilateral upper extremity (e.g., Pancoast tumors). Lung cancer is associated with a variety of paraneoplastic syndromes: (i) most of such paraneoplastic syndromes are associated with small cell lung cancer; (ii) squamous cell carcinomas are more likely to be associated with hypercalcemia due to parathyroidlike hormone production; and (iii) clubbing and hypertrophic pulmonary osteoarthropathy and the Trousseau syndrome of hypercoagulability are caused more frequently by adenocarcinomas. Eaton-Lambert myasthenic syndrome is reported in association with small cell and non-small cell lung cancers. Paraneoplastic syndromes can pose debilitating problems in cancer patients and can complicate the medical management of such patients.

Non-small cell lung cancer (NSCLC) accounts for nearly 80% of lung cancer, and surgically resectable cases account for less than 30%. Chemotherapy and radiotherapy are tried in unresectable cases, but the median survival period is only 15-20 months and the 3-year survival rate is approximately 30-40% in stage IIIA and IIIB cases. The prognosis is even worse in stage IV patients with a median survival period of 8-10 months and a 1-year survival rate of less than 30%. At these advanced stages, the main therapeutic objectives are increasing the survival period and symptomatic relief. *See, e.g.,* Cortes-Funes H., New Treatment Approaches for Lung Cancer and Impact on Survival. Semin. Oncol. 29:26-29 (2002); Fukuoka, M and Saijoh, N., Practical medicine -Lung cancer, Nannkodo (2001).

NSCLC is pathologically characterized further into adenocarcinoma, squamous cell carcinoma, large cell carcinoma, and other less common forms. Clinically there are also important differences in NSCLC that can be observed in smokers and non-smokers.

A summary of clinical characteristics by histologic NSCLC subtype include:
- Adenocarcinoma is the most frequent non-small cell lung cancer (NSCLC) in the United States, representing 35-40% of all lung cancers, usually occurring in a peripheral location within the lung and arising from bronchial mucosal glands. Adenocarcinoma is the most common histologic subtype, manifesting as a scar carcinoma. This is the subtype observed most commonly in persons who do not smoke, however, adenocarcinoma is also common in smokers. This type of NSCLC may also manifest as multifocal tumors in a bronchoalveolar form. Bronchoalveolar carcinoma is a distinct subtype of adenocarcinoma with the classic manifestation as an interstitial lung disease upon radiographic imaging. Bronchoalveolar carcinoma arises from type II pneumocytes and grows along alveolar septa. This subtype may manifest as a solitary peripheral nodule, multifocal disease, or a rapidly progressing pneumonic form. A characteristic finding in persons with advanced disease is voluminous watery sputum.
- Squamous cell carcinoma accounts for 25-30% of all lung cancers. The classic manifestation is a cavitary lesion in a proximal bronchus. This type is characterized histologically by the presence of keratin pearls and can be detected based on results from cytologic studies because it has a tendency to exfoliate. It is the type most often associated with hypercalcemia.
- Large cell carcinoma accounts for 10-15% of lung cancers, typically manifesting as a large peripheral mass upon radiographic imaging. Histologically, this type has sheets of highly atypical cells with focal necrosis, with no evidence of keratinization (typical of squamous cell carcinoma) or gland formation (typical of adenocarcinomas). Patients with large cell carcinoma are more likely to develop gynecomastia and galactorrhea as paraneoplastic syndromes.

### II. Adenocarcinoma

Adenocarcinoma is a cancer that originates in glandular tissue. Glandular tissue comprises organs that synthesize a substance for release such as hormones. Glands can be divided into two general groups: (i) endocrine glands - glands that secrete their product directly onto a surface rather than through a duct, often into the blood stream and (ii) exocrine glands - glands that secrete their products via a duct, often into cavities inside the body or its outer surface. Exocrine glands may be further differentiated into three categories: apocrine, holocrine, and merocrine. However, it should be noted that to be classified as adenocarcinoma, the cells do not necessarily need to be part of a gland, as long as they have secretory properties. Adenocarcinoma may be derived from various tissues including, but not limited to, breast, colon, lung, prostate, salivary gland, stomach, liver, gall bladder, pancreas (99% of pancreatic cancers are ductal adenocarcinomas), cervix, vagina, and uterus, as well as unknown primary adenocarcinomas.

Adenocarcinoma is a neoplasm which frequently presents marked difficulty in differentiating from where and from which type of glandular tissue the tumor(s) arose. Thus, an adenocarcinoma identified in the lung may have had its origins (or may have metastasized) from an ovarian adenocarcinoma. Cancer for which a primary site cannot be found is called cancer of unknown primary. The primary site is identified, after the initial diagnosis of carcinoma, in only approximately 10-20% of patients during their remaining life times and it frequently is not identified until post-mortem examination. It has been reported that approximately 60% of patients *(i.e.,* over 50,000 patients per annum in the United States) who are diagnosed with carcinoma of unknown primary site suffer from adenocarcinoma.

A diagnosis of adenocarcinoma which is not further described *(i.e.,* adenocarcinoma not otherwise specified; adenocarcinoma NOS) is often a preliminary diagnosis and can frequently be clarified with the use of immunohistochemistry or fluorescent *in situ* hybridization (FISH) (*see, e.g.,* Dabbs, D.J. and Silverman, J.F., Immunohistochemical and Fluorescent in situ Hybridization Workup of Metastatic Carcinoma of Unknown Primary. Path. Case Rev. 6(4): 146-153 (2005)), and/or various imaging methodologies including, but not limited to, computerized tomography (CT), magnetic resonance imaging (MRI), and positron emission tomography (PET).

Immunohistochemistry refers to the process of localizing proteins in cells of a tissue section exploiting the principle of antibodies binding specifically to antigens in biological tissues. Immunohistochemistry is also widely used in basic research to understand the distribution and localization of biomarkers in different parts of a tissue. Immunohistochemical staining is widely used specialized technique in the diagnosis of cancer and the classification of neoplasms. The antibodies utilized may be either polyclonal or monoclonal in nature and may be directed against cell components or products which can include: (i) enzymes (e.g., prostatic acid phosphatase, neuron-specific enoenzymes); (ii) normal tissue components (e.g., keratin, neurofilaments); and (iii) hormones or hormone receptors (e.g., estrogen receptor, oncofetal antigens, S-100 proteins). It should be noted that specific molecular markers are characteristic of particular cancer types. For example, adenocarcinoma often gives positive immunohistochemical results for thyroid transcription factor-1 (TTF-1). Visualizing an antibody-antigen interaction can be accomplished in a number of ways. In the most common instance, an antibody is conjugated to an enzyme, such as peroxidase, that can catalyze a color-producing reaction, as with immunoperoxidase staining. Alternatively, the antibody can also be tagged to a fluorophore, such as FITC, rhodamine, Texas Red, or DyLight Fluor, as with immunofluorescence.

Fluorescent *in situ* hybridization (FISH) is a cytogenetic technique that can be used to detect and localize the presence or absence of specific DNA sequences on chromosomes. It utilizes fluorescent-tagged nucleic acid probes that bind to only those parts of the chromosome with which they show a high degree of nucleotide sequence complmentarity. Fluorescence microscopy can be used to find out where the fluorescent probe bound to the chromosome.

As set forth above, non-small cell lung carcinoma (NSCLC) and adenocarcinoma are highly prevalent forms of cancer and account for a large percentage of the deaths associated with cancer world-wide. Given the relatively refractory nature of NSCLC and adenocarcinoma to many forms of therapy there remains an, as yet unmet, need for the development of compositions and treatment regimens that are both generally safe and effective for increasing the survival of patients receiving chemotherapy, slowing the progression of their tumors, and/or stimulating or maintaining the beneficial physiological function of important bodily processes in normal *(i.e.,* non-cancerous) cells and tissues.

In addition to the foregoing considerations regarding cancer, many patients, including cancer patients receiving chemotherapy, are also in need of: maintaining or stimulating hematological function; maintaining or stimulating erythropoietin function or synthesis; mitigating or preventing anemia; and maintaining or stimulating pluripotent, multipotent, and unipotent normal stem cell function or synthesis.

US 6037336 discloses the use of dimesna in the treatment of carboplatin-induced toxicity.

### SUMMARY OF THE INVENTION

The present invention 2,2'-dithio-bis-ethane sulfonate or a pharmaceutically acceptable salt thereof, for use in mitigating or preventing chemotherapy-induced anemia in a patient, wherein the patient suffers from lung cancer or adenocarcinoma and is treated with a taxane medicament and/or cisplatin.

In brief, there are disclosed herein: (i) compositions, methods, and kits which lead to an increase in patient survival time in cancer patients receiving chemotherapy; (ii) compositions and methods which cause cytotoxic or apoptotic potentiation of the anti-cancer activity of chemotherapeutic agents; (iii) compositions and methods for maintaining or stimulating hematological function in patients in need thereof, including those patients suffering from cancer; (iv) compositions and methods for maintaining or stimulating erythropoietin function or synthesis in patients in need thereof, including those patients suffering from cancer; (v) compositions and methods for mitigating or preventing anemia in patients in need thereof, including those patients suffering from cancer; (vi) compositions and methods for maintaining or stimulating pluripotent, multipotent, and unipotent normal stem cell function or synthesis in patients in need thereof, including those patients suffering from cancer; (vii) compositions and methods which promote the arrest or retardation of tumor progression in cancer patients receiving chemotherapy; (viii) compositions and methods for increasing patient survival and/or delaying tumor progression while maintaining or improving the quality of life in a cancer patient receiving chemotherapy; (ix) novel methods of the administration of taxane and platinum medicaments and a Formula (I) compound of the present invention to a cancer patient; and (x) kits to achieve one or more of the aforementioned physiological effects in a patient in need thereof, including those patients suffering from cancer.

In one aspect of the disclosure, a patient suffering from lung cancer treated with taxane and/or platinum medicaments is given a medically sufficient dosage of a Formula (I) compound so as to increase patient survival time in said patient suffering from lung cancer.

In another aspect, the lung cancer is non-small cell lung carcinoma.

In another aspect, the increase in patient survival time in said patient suffering from lung cancer and treated with a Formula (I) compound is expected to be at least 30 days longer than the expected survival time if said patient was not treated with a Formula (I) compound.

In yet another aspect, a patient suffering from lung cancer was treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, a patient suffering from lung cancer was treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from lung cancer were male or female and smokers or non-smokers.

In one aspect of the disclosure, a patient suffering from adenocarcinoma treated with taxane and/or platinum medicaments is given a medically sufficient dosage of a Formula (I) compound so as to increase patient survival time in said patient suffering from adenocarcinoma.

In another aspect, the increase in patient survival time in said patient suffering from adenocarcinoma and treated with a Formula (I) compound is expected to be at least 30 days longer than the expected survival time if said patient was not treated with a Formula (I) compound.

In yet another aspect, a patient suffering from adenocarcinoma is treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, a patient suffering from adenocarcinoma is treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from adenocarcinoma were male or female and smokers or non-smokers.

In one aspect of the disclosure, a patient suffering from lung cancer treated with taxane and platinum medicaments is given a medically sufficient dosage of a Formula (I) compound so as to potentiate the chemotherapeutic effect in said patient suffering from lung cancer.

In another aspect, the lung cancer is non-small cell lung carcinoma.

In yet another aspect, the chemotherapeutic effect is potentiated in a patient suffering from lung cancer treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, the chemotherapeutic effect is potentiated in a patient suffering from lung cancer treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from lung cancer were male or female and smokers or non-smokers.

In one aspect of the disclosure, the chemotherapeutic effect is potentiated in a patient suffering from adenocarcinoma who is treated with taxane and platinum medicaments and is also given a medically sufficient dosage of a Formula (I) compound so as to increase patient survival time in said patient suffering from adenocarcinoma.

In yet another aspect, the chemotherapeutic effect is potentiated in a patient suffering from adenocarcinoma treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, the chemotherapeutic effect is potentiated in a patient suffering from adenocarcinoma treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from adenocarcinoma were male or female and smokers or non-smokers.

In one aspect of the disclosure, hematological function is maintained or stimulated in a patient in need thereof, by providing to said patient a composition comprised of a Formula (I) compound in a medically sufficient dosage.

In one aspect, a patient suffering from lung cancer treated with taxane and/or platinum medicaments is given a medically sufficient dosage of a Formula (I) compound so as to maintain or stimulate hematological function in said patient suffering from lung cancer.

In another aspect, the lung cancer is non-small cell lung carcinoma.

In yet another aspect, the hematological function is maintained or stimulated in a patient suffering from lung cancer treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, the hematological function is maintained or stimulated in a patient buffering from lung cancer treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from lung cancer were male or female and smokers or non-smokers.

In one aspect of the disclosure, the hematological function is maintained or stimulated in a patient suffering from adenocarcinoma who is treated with taxane and/or platinum medicaments and is also given a medically sufficient dosage of a Formula (I) compound so as to maintain or stimulate hematological function in said patient suffering from adenocarcinoma.

In yet another aspect, the hematological function is maintained or stimulated in a patient suffering from adenocarcinoma treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, the hematological function is maintained or stimulated in a patient suffering from adenocarcinoma treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from adenocarcinoma were male or female and smokers or non-smokers.

In one aspect of the disclosure, erythropoietin function or synthesis or homeostatic function of erythropoiesis is maintained or stimulated in a patient in need thereof, by providing to said patient a composition comprised of a Formula (I) compound in a medically sufficient dosage.

In one aspect of the disclosure, a patient suffering from lung cancer treated with taxane and/or platinum medicaments is given a medically sufficient dosage of a Formula (I) compound so as to maintain or stimulate erythropoietin function or synthesis or homeostatic function of erythropoiesis in said patient suffering from lung cancer.

In another aspect, the lung cancer is non-small cell lung carcinoma.

In yet another aspect, the erythropoietin function or synthesis or homeostatic function of erythropoiesis is maintained or stimulated in a patient suffering from lung cancer treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, the erythropoietin function or synthesis or homeostatic function of erythropoiesis is maintained or stimulated in a patient suffering from lung cancer treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from lung cancer were male or female and smokers or non-smokers.

In one aspect of the disclosure, the erythropoietin function or synthesis or homeostatic function of erythropoiesis is maintained or stimulated in a patient suffering from adenocarcinoma who is treated with taxane and/or platinum medicaments and is also given a medically sufficient dosage of a Formula (I) compound so as to maintain or stimulate erythropoietin function or synthesis or homeostatic function of erythropoiesis in said patient suffering from adenocarcinoma.

In yet another aspect, the erythropoietin function or synthesis or homeostatic function of erythropoiesis is maintained or stimulated in a patient suffering from adenocarcinoma treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, the erythropoietin function or synthesis or homeostatic function of erythropoiesis is maintained or stimulated in a patient suffering from adenocarcinoma treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from adenocarcinoma were male or female and smokers or non-smokers.

In one embodiment, anemia is mitigated or prevented in a patient in need thereof, by providing to said patient a composition comprised of the claimed Formula (I) compound in a medically sufficient dosage.

In one embodiment, a patient suffering from lung cancer treated with taxane and/or platinum medicaments is given a medically sufficient dosage of the claimed Formula (I) compound so as to mitigate or prevent chemotherapy-induced anemia in said patient suffering from lung cancer.

In another embodiment, the lung cancer is non-small cell lung carcinoma.

In yet another embodiment, chemotherapy-induced anemia is mitigated or prevented in a patient suffering from lung cancer treated with paclitaxel, the claimed

Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another embodiment, chemotherapy-induced anemia is mitigated or prevented in a patient suffering from lung cancer treated with paclitaxel, the claimed Formula (I) compound, and cisplatin once every 3 weeks, wherein the dose of paclitaxel was approximately 175 mg/m², the dose of the claimed Formula (I) compound was approximately 18.4 g/m², and the dose of cisplatin ranged from 75 mg/m² to 85 mg/m², wherein said administration of paclitaxel, the claimed Formula (I) compound, and cisplatin once every 3 weeks was repeated for 6 cycles.

In another embodiment, the patients suffering from lung cancer were male or female and smokers or non-smokers.

In one embodiment, chemotherapy-induced anemia is mitigated or prevented in a patient suffering from adenocarcinoma who is treated with taxane and/or platinum medicaments and is also given a medically sufficient dosage of the claimed Formula (I) compound so as to mitigate or prevent chemotherapy-induced anemia.

In yet another embodiment, chemotherapy-induced anemia is mitigated or prevented in a patient suffering from adenocarcinoma treated with paclitaxel, the claimed Formula (I) compound, and cisplatin once every 2-4 weeks, wherein the dose of paclitaxel ranged from 160 mg/m² to 190 mg/m², the dose of the claimed Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another embodiment, chemotherapy-induced anemia is mitigated or prevented in a patient suffering from adenocarcinoma treated with paclitaxel, the claimed Formula (I) compound, and cisplatin once every 3 weeks, wherein the dose of paclitaxel was approximately 175 mg/m², the dose of the claimed Formula (I) compound was approximately 18.4 g/m², and the dose of cisplatin ranged from 75 mg/m² to 85 mg/m², wherein said administration of paclitaxel, the claimed Formula (I) compound, and cisplatin once every 3 weeks was repeated for 6 cycles.

In another embodiment, the patients suffering from adenocarcinoma were male or female and smokers or non-smokers.

In one aspect of the disclosure, pluripotent, multipotent, and unipotent normal stem cell function or synthesis is maintained or stimulated in a patient in need thereof, by providing to said patient a composition comprised of the claimed Formula (I) compound in a medically sufficient dosage.

In one aspect, a patient suffering from lung cancer treated with taxane and/or platinum medicaments is given a medically sufficient dosage of a Formula (I) compound so as to maintain or stimulate pluripotent, multipotent, and unipotent normal stem cell function or synthesis in said patient suffering from lung cancer.

In another aspect, the lung cancer is non-small cell lung carcinoma.

In yet another aspect, pluripotent, multipotent, and unipotent normal stem cell function or synthesis is maintained or stimulated in a patient suffering from lung cancer treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, pluripotent, multipotent, and unipotent normal stem cell function or synthesis is maintained or stimulated in a patient suffering from lung cancer treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from lung cancer were male or female and smokers or non-smokers.

In one aspect of the disclosure, pluripotent, multipotent, and unipotent normal stem cell function or synthesis is maintained or stimulated in a patient suffering from adenocarcinoma who is treated with taxane and/or platinum medicaments and is also given a medically sufficient dosage of a Formula (I) compound so as to maintain or stimulate pluripotent, multipotent, and unipotent normal stem cell function or synthesis in said patient suffering from adenocarcinoma.

In yet another aspect, pluripotent, multipotent, and unipotent normal stem cell function or synthesis is maintained or stimulated in a patient suffering from adenocarcinoma treated with paclitaxel, a Formula (I) compound, and cisplatin once every 2-4 weeks, wherein the dose of paclitaxel ranged from 160 mg/m² to 190 mg/m², the dose of a Formula (I) compound ranged from 14 g/m² to 22 g/m², and the dose of cisplatin ranged from 60 mg/m² to 100 mg/m², wherein said administration of paclitaxel, a Formula (I) compound, and cisplatin once every 2-4 weeks was repeated at least once.

In still another aspect, pluripotent, multipotent, and unipotent normal stem cell function or synthesis is maintained or stimulated in a patient suffering from adenocarcinoma treated with paclitaxel, a Formula (I) compound, and cisplatin once every 3 weeks, wherein the dose of paclitaxel was approximately 175 mg/m², the dose of a Formula (I) compound was approximately 18.4 g/m², and the dose of cisplatin ranged from 75 mg/m² to 85 mg/m², wherein said administration of paclitaxel, a Formula (I) compound, and cisplatin once every 3 weeks was repeated for 6 cycles.

In another aspect, the patients suffering from adenocarcinoma were male or female and smokers or non-smokers.

In another aspect, the Formula (I) compounds increase patient survival and/or delay tumor progression while maintaining nr improving the quality of life of said patients diagnosed with lung cancer who are being treated with the taxane and/or platinum medicaments of the present invention.

In another aspect, the lung cancer is non-small cell lung carcinoma.

In another aspect, the Formula (I) compounds increase patient survival and/or delay tumor progression while maintaining or improving the quality of life of said patients diagnosed with adenocarcinoma who are being treated with the taxane and/or platinum medicaments of the present invention.

In another aspect, the patients suffering from adenocarcinoma were male or female and smokers or non-smokers.

In another aspect, the platinum medicaments of the present invention include cisplatin, oxaliplatin, carboplatin, satraplatin, and derivatives and analogs thereof.

In another aspect, the taxane medicament is selected from the group consisting of docetaxel, paclitaxel, paclitaxel derivatives, polyglutamylated forms of paclitaxel, liposomal paclitaxel, and derivatives and analogs thereof.

In still another aspect, the compositions of Formula (I) include 2,2'-dithio-bis-ethane sulfonate, a pharmaceutically-acceptable salt thereof, and/or an analog thereof, as well as prodrugs, analogs, conjugates, hydrates, solvates and polymorphs, as well as stereoisomers (including diastereoisomers and enantiomers) and tautomers of such compounds.

In still another aspect, the dose rate of the taxane and platinum medicaments ranged from 10-20 mg/m²/day and the dose rate of a Formula (I) compound ranged from 4.1-41.0 g/m² per day; the concentration of the taxane and platinum medicaments and/or Formula (I) compounds is at least 0.01 mg/mL; the infusion time of the taxane and platinum medicaments and/or Formula (I) compounds is from 5 minutes to 24 hours, and can be repeated as needed and tolerated in a given patient; the schedule of administration of the taxane and platinum medicaments and/or Formula (I) compounds is every 2-8 weeks.

In another aspect of the disclosure, a kit comprising a Formula (I) compound for administration to a patient, and instructions for administering said Formula (I) compound in an amount sufficient to cause one or more of the physiological effects selected from the group consisting of: increasing patient survival time of said cancer patient receiving taxane and platinum medicaments; causing a cytotoxic or apoptotic potentiation of the chemotherapeutic effects of said taxane and platinum medicaments; maintaining or stimulating hematological function in said patient, including said patient with cancer receiving chemotherapy; maintaining or stimulating erythropoietin function or synthesis in said patient, including said patient with cancer receiving chemotherapy; mitigating or preventing anemia in said patient, including said patient with cancer receiving chemotherapy; maintaining or stimulating pluripotent, multipotent, and unipotent normal stem cell function or synthesis in said patient, including said patient with cancer receiving chemotherapy; promoting the arrest or retardation of tumor progression in said cancer patient receiving taxane and/or platinum medicaments; and/or increasing patient survival and/or delaying tumor progression while maintaining or improving the quality of life in said cancer patient receiving taxane and platinum medicaments.

In another aspect, the cancer patient has lung cancer.

In yet another aspect, the lung cancer is non-small cell lung cancer.

In still another aspect, the cancer patient has an adenocarcinoma.

In one aspect, the kit further contains instructions for administering a taxane medicament and a platinum medicament selected from the group consisting of cisplatin, oxaliplatin, carboplatin, satraplatin, and derivatives and analogs thereof.

In another aspect, the kit further contains instructions for administering a platinum medicament and a taxane medicament selected from the group consisting of docetaxel, paclitaxel, polyglutamylated forms of paclitaxel, liposomal paclitaxel, and derivatives and analogs thereof.

In yet another aspect, the platinum and taxane medicaments are cisplatin and paclitaxel.

### DESCRIPTION OF THE FIGURES

**Fig. 1** illustrates, in tabular form, the Primary Endpoint *(i.e.,* the mitigation or prevention of patient peripheral neuropathy) of the Japan Phase III Clinical Trial supporting the present invention as determined utilizing the Peripheral Neuropathy Questionnaire (PNQ^{©}).
**Fig. 2** illustrates, in tabular form, an evaluation of the statistical power observed in the Japan Phase III Clinical Trial with respect to the Primary Endpoint *(i.e.,* the mitigation or prevention of patient peripheral neuropathy), as measured by the Generalized Estimating Equation (GEE) method.
**Fig. 3** illustrates, in tabular form, a Secondary Endpoint (*i.e.*, a decrease in patient hemoglobin, erythrocyte, and hematocrit levels) of the Japan Phase III Clinical Trial supporting the present invention, in patient populations receiving Tavocept™ (BNP7787) or placebo.
**Fig. 4** illustrates, in tabular form, a Secondary Endpoint *(i.e.,* tumor response rate to chemotherapy administration) of the Japan Phase III Clinical Trail supporting the present invention, in patient populations receiving either Tavocept™ (BNP7787) or placebo, as measured by the physician or by the Independent Radiological Committee (IRC) criteria.
**Fig. 5** illustrates, in graphical form, a Secondary Endpoint (*i.e*., patient survival) of the Japan Phase III Clinical Trial supporting the present invention, in patient populations diagnosed with non-small cell lung carcinoma receiving either Tavocept™ (BNP7787) or placebo.
**Fig. 6** illustrates, in graphical form, a Secondary Endpoint (*i.e*., patient survival) of the Japan Phase III Clinical Trial supporting the present invention, in female patient populations receiving either Tavocept™ (BNP7787) or placebo.
**Fig. 7** illustrates, in graphical form, a Secondary Endpoint (*i.e*., patient survival) of the Japan Phase III Clinical Trial supporting the present invention, in patient populations diagnosed with adenocarcinoma receiving either Tavocept™ (BNP7787) or placebo.

### DETAILED DESCRIPTION OF THE INVENTION

### DEFINITIONS

As utilized herein, the term "administration" with respect to the taxane and platinum medicaments and Formula (I) compounds of the present invention includes administering, providing, or giving such medicaments or compounds to a patient by one or more of the following means: oral, topical, parenteral (e.g., intravenous, intraarterial, intratumoral, and peritumoral), and *per* rectum.

As utilized herein, the definitions for the terms "Adverse Event" (effect or experience), "Adverse Reaction", and unexpected adverse reaction have previously been agreed to by consensus of the more than 30 Collaborating Centers of the WHO International Drug Monitoring Centre (Uppsala, Sweden). *See,* Edwards, I.R., et al., Harmonisation in Pharmacovigilance Drug Safety 10(2):93-102 (1994). The following definitions, with input from the WHO Collaborative Centre, have been agreed to:
1. Adverse Event (Adverse Effect or Adverse Experience) - Any untoward medical occurrence in a patient or clinical investigation subject administered a pharmaceutical product and which does not necessarily have to have a causal relationship with this treatment. An Adverse Event (AE) can therefore be any unfavorable and unintended sign (including an abnormal laboratory finding, for example), symptom, or disease temporally associated with the use of a medicinal product, whether or not considered related to the medicinal product.
2. Adverse Drug Reaction (ADR) - In the *pre-approval clinical experience* with a new medicinal product or its new usages, particularly as the therapeutic dose(s) may not be established: all noxious and unintended responses to a medicinal product related to any dose should be considered adverse drug reactions. Drug-related Adverse Events are rated from grade 1 to grade 5 and relate to the severity or intensity of the event. Grade 1 is mild, grade 2 is moderate, grade 3 is severe, grade 4 is life threatening, and grade 5 results in the subject's death.
3. Unexpected Adverse Drug Reaction - An adverse reaction, the nature or severity of which is not consistent with the applicable product information.
Serious Adverse Event or Adverse Drug Reaction: A Serious Adverse Event (experience or reaction) is any untoward medical occurrence that at any dose:
(i) Results in death or is life-threatening. It should be noted that the term "life-threatening" in the definition of "serious" refers to an event in which the patient was at risk of death at the time of the event; it does not refer to an event which hypothetically might have caused death if it were more severe.
(ii) Requires inpatient hospitalization or prolongation of existing hospitalization.
(iii) Results in persistent or significant disability/incapacity, or
(iv) Is a congenital anomaly/birth defect.

As utilized herein the term "cancer" refers to all known forms of cancer including, solid forms of cancer (e.g., tumors), lymphomas, and leukemias.

As utilized herein, the term "clinical trial" or "trial", refers to the Japan Phase III Clinical Trial disclosed in the present invention which was utilized to show the ability of Tavocept™ (also referred to in the literature as disodium 2,2'-dithio-bis-ethane sulfonate, dimesna, or BNP7787) to prevent and/or reduce peripheral neuropathy induced by paclitaxel/cisplatin combination therapy. The incidence and severity of adverse reactions, time to their onset, etc. and the like, were compared between patients treated with Tavocept™ and those given a placebo using Quality of Life (QOL) questionnaires *(i.e.,* Peripheral Neuropathy Questionnaire (PNQ^{©}) and CIPN-20)) and the National Cancer Institute - Common Toxicity Criteria (NCI-CTC). The effects of Tavocept™ on the Quality of Life (QOL) of patients under anticancer treatment was also evaluated using the QOL questionnaire, EORTC QLQ-C30. Whether or not Tavocept™ would affect the efficacy of paclitaxel/cisplatin combination therapy was also evaluated based on the response rate, aggravation-free survival period, and total survival period. In order to make all these evaluations, Tavocept™ (approximately14-22 g/m², most preferably approximately 18.4 g/m²) or placebo (0.9% NaCl) was administered to non-small cell lung carcinoma (NSCLC) patients, including adenocarcinoma patients, under chemotherapy with paclitaxel (approximately 160-190 mg/m², most preferably approximately 175 mg/m²) and cisplatin (approximately 60-100 mg/m², most preferably approximately 80 mg/m²), every 3 weeks (and repeated for a minimum of 2 cycles).

As utilized herein, adenocarcinoma refers to a cancer that originates in glandular tissue. Glandular tissue comprises organs that synthesize a substance for release such as hormones. Glands can be divided into two general groups: (i) endocrine glands - glands that secrete their product directly onto a surface rather than through a duct, often into the blood stream and (ii) exocrine glands - glands that secrete their products via a duct, often into cavities inside the body or its outer surface. However, it should be noted that to be classified as adenocarcinoma, the tissues or cells do not necessarily need to be part of a gland, as long as they have secretory properties. Adenocarcinoma may be derived from various tissues including, but not limited to, breast, colon, lung, prostate, salivary gland, stomach, liver, gall bladder, pancreas (99% of pancreatic cancers are ductal adenocarcinomas), cervix, vagina, and uterus, as well as unknown primary adenocarcinomas. Adenocarcinoma is a neoplasm which frequently presents marked difficulty in differentiating from where and from which type of glandular tissue the tumor(s) arose. Thus, an adenocarcinoma identified in the lung may have had its origins (or may have metastasized) from an ovarian adenocarcinoma. Cancer for which a primary site cannot be found is called cancer of unknown primary.

As utilized herein, the term non-small cell lung cancer (NSCLC) accounts for approximately 75% of all primary lung cancers. NSCLC is pathologically characterized further into adenocarcinoma, squamous cell carcinoma, large cell carcinoma, and other less common forms. Clinically there are important differences in NSCLC that can be observed in smokers and non-smokers.

As used herein, the term "potentiate", "potentiating", "chemotherapy potentiating", "chemotherapeutic effect is potentiated", and "potentiating the chemotherapeutic effects" is defined herein as producing one or more of the following physiological effects: (i) the increase or enhancement of the cytotoxic activity of chemotherapy agents by acting in an additive or synergistic cytotoxic manner with said chemotherapeutic agents within the tumor cells; (ii) reducing, preventing, mitigating, and/or delaying said deleterious physiological manifestations of said cancer in subjects suffering therewith; (iii) selectively sensitizing cancer cells to the anti-cancer activity of chemotherapeutic agents; and/or (iv) restoring apoptotic effects or sensitivity in tumor cells.

As used herein, the term "chemotherapeutic agent" or "chemotherapy agent" refer to an agent that reduces, prevents, mitigates, limits, and/or delays the growth of metastases or neoplasms, or kills neoplastic cells directly by necrosis or apoptosis of neoplasms or any other mechanism, or that can be otherwise used, in a pharmaceutically-effective amount, to reduce, prevent, mitigate, limit, and/or delay the growth of metastases or neoplasms in a subject with neoplastic disease. Chemotherapeutic agents include, for example, fluropyrimidines; pyrimidine nucleosides; purine nucleosides; anti-folates, platinum complexes; anthracyclines/anthracenediones; epipodophyllotoxins; camptothecins; hormones; hormonal complexes; antihormonals; enzymes, proteins, peptides and polyclonal and/or monoclonal antibodies; vinca alkaloids; taxanes; epothilones; antimicrotubule agents; alkylating agents; antimetabolites; topoisomerase inhibitors; antivirals; and various other cytotoxic and cytostatic agents.

As used herein, the term "cytostatic agents" are mechanism-based agents that slow the progression of neoplastic disease.

As used herein the term "cytotoxic agents" are any agents or processes that kill neoplastic cells.

As utilized herein, the term "chemotherapeutic effect" refers to the ability of an agent to reduce, prevent, mitigate, limit, and/or delay the growth of metastases or neoplasms, or kill neoplastic cells directly by necrosis or apoptosis of neoplasms or any other mechanism, or that can be otherwise used to reduce, prevent, mitigate, limit, and/or delay the growth of metastases or neoplasms in a subject with neoplastic disease.

As used herein, the term "platinum medicaments" or "platinum compounds" include all compounds, compositions, and formulations which contain a platinum ligand in the structure of the molecule. By way of non-limiting example, the valence of the platinum ligand contained therein may be platinum II or platinum IV. The platinum medicaments or platinum compounds of the present invention include, in a non-limiting manner, cisplatin, oxaliplatin, carboplatin, satraplatin, and analogs and derivatives thereof.

At used herein, the term "taxane medicaments" included, in a noun-limiting manner, docetaxel or paclitaxel (including the commercially-available paclitaxel derivatives Taxol^{®} and Abraxane^{®}), polyglutamylated forms of paclitaxel (e.g., Xyotax^{®}), liposomal paclitaxel (*e.g*.,Tocosol^{®}), and analogs and derivatives thereof.

As utilized herein, the term "chemotherapy" or "chemotherapeutic regimen(s)" refers to treatment using the above-mentioned chemotherapeutic agents with or without the Formula (I) compounds of the present invention.

As utilized herein, the term "colony-stimulating factor" (CSF) are secreted glycoproteins which bind to receptor proteins on the surfaces of hematopoietic stem cells and thereby activate intracellular signaling pathways which can cause the cells to proliferate and differentiate into a specific kind of blood cell (usually white blood cells). Hematopoietic stem cells (HSC) are stem cells *(i.e.,* cells retain the ability to renew themselves through mitotic cell division and can differentiate into a diverse range of specialized cell types) that give rise to all the blood cell types including myeloid (e.g., monocytes, macrophages, neutrophiles, basophils, eosinophils, erythrocytes, megakaryocytes/platelets, dendritic cells, and the like) and lymphoid lineages (e.g., T-cells, B-cells, NK-cells, and the like). Colony-stimulating factors include: macrophage colony-stimulating factor (CSF-1); granulocyte-macrophage colony-stimulating factor (CSF-2); and granulocyte colony-stimulating factor (CSF-3).

As utilized herein, the term "cycle" refers to the administration of a complete regimen of medicaments to the patient in need thereof in a defined time period. For example, in the Japan Phase III Clinical Trial disclosed herein, a cycle would comprise the administration of taxane and platinum medicaments, a Formula (I) compound, and any associated medications which may be required (*e.g*., pre-hydration, anti-emesis drugs, and the like) to the patient within a defined time period.

As used herein the term "erythropoiesis" refers to the process by which red blood cells (erythrocytes) are produced. In the early fetus, erythropoiesis takes place in the mesodermal cells of the yolk sac. By the third or fourth month of fetal development, erythropoiesis moves to the spleen and liver. In human adults, erythropoiesis generally occurs within the bone marrow. The long bones of the arm (tibia) and leg (femur) cease to be important sites of hematopoiesis by approximately age 25; with the vertebrae, sternum, pelvis, and cranial bones continuing to produce red blood cells throughout life. However, it should be noted that in humans with certain diseases and in some animals, erythropoiesis also occurs outside the bone marrow, within the spleen or liver. This is termed extramedullary erythropoiesis. In the process of red blood cell maturation, a cell undergoes a series of differentiations. The following stages of development all occur within the bone marrow: (i) pluripotent hematopoietic stem cell; (ii) multipotent stem cell; (iii) unipotent stem cell; (iv) pronormoblast; (v) basophilic normoblast/early normoblast; (vi) polychrmatophilic normoblast/intermediate normoblast; (vii) orthochromic normoblast/late normoblast; and (viii) reticulocyte. Following these stages, the cell is released from the bone marrow, and ultimately becomes an "erythrocyte" or mature red blood cell circulating in the peripheral blood.

As used herein, the term "erythropoietin" is a glycoprotein hormone that is a cytokine for erythrocyte (red blood cell) precursors in the bone marrow which regulates the process of red blood cell production *(i.e.,* erythropoiesis). Erythropoietin (EPO) is produced mainly by peritubular fibroblasts of the renal cortex. Regulation is believed to rely on a feed-back mechanism measuring blood oxygenation. Constitutively synthesized transcription factors for EPO, known as hypoxia inducible factors (HIFs), are hydroxylized and proteosomally-digested in the presence of oxygen.

As used herein, the term "Formula (I) compound" or "Formula (I) composition" means 2,2'-dithio-bis-ethane sulfonate and pharmaceutically-acceptable salts thereof. Specifically included, in a non-limiting manner, in the term "Formula (I) compound" or "Formula (I) composition" is disodium 2,2'-dithio-bis-ethane sulfonate (also known in the literature as dimesna, BNP7787, and Tavocept™). Also included, is the key metabolite of disodium 2,2'-dithio-bis-ethane sulfonate, 2-mercapto ethane sulfonate sodium (also known in the literature as mesna). Various compounds of Formula (I), and their synthesis are described in, *e.g.*, published U.S. Patent Application No. 2005/0256055.

As used herein, the term "a medically sufficient dosage" in reference to the compounds or compositions of the instant invention refers to the dosage that is sufficient to induce a desired biological, pharmacological, or therapeutic outcome in a subject with need thereof.

As used herein the term "g/m² " represents the amount of a given compound or formulation in grams per square meter of the total body surface area of the subject to whom the compound or formulation is administered.

As used herein the term "mg/m²" represents the amount of a given compound or formulation in milligrams per square meter of the total body surface area of the subject to whom the compound or formulation is administered.

"Nucleophile" means an ion or molecule that donates a pair of electrons to an atomic nucleus to form a covalent bond; the nucleus that accepts the electrons is called an electrophile. This occurs, for example, in the formation of acids and bases according to the Lewis concept, as well as in covalent carbon bonding in organic compounds.

As utilized herein, the term "patient" refers to any individual or subject, without limitation, who is in need of treatment with a compound, composition, medicament, formulation, method, or kit which is disclosed in the present invention.

"Pharmaceutically-acceptable salt" means salt derivatives of drugs which are accepted as safe for human administration. In the present invention, the Formula (I) compounds of the present invention include pharmaceutically-acceptable salts, which include but are not limited to: (i) a monosodium salt; (ii) a disodium salt; (iii) a sodium potassium salt; (iv) a dipotassium salt; (v) a calcium salt; (vi) a magnesium salt; (vii) a manganese salt; (viii) an ammonium salt; and (ix) a monopotassium salt.

As used herein the term "Quality of Life" or "QOL" refers, in a non-limiting manner, to a maintenance or increase in a cancer patient's overall physical and mental state (e.g., cognitive ability, ability to communicate and interact with others, decreased dependence upon analgesics for pain control, maintenance of ambulatory ability, maintenance of appetite and body weight (lack of cachexia), lack of or diminished feeling of "hopelessness"; continued interest in playing a role in their treatment, and other similar mental and physical states).

As used herein, the term "reducing" includes preventing, attenuating the overall severity of, delaying the initial onset of, and/or expediting the resolution of the acute and/or chronic condition suffered by the patient.

As used herein, the term "treat" or "treated", with respect to a patient *without* cancer, refers to a patient, who is in need thereof, and who has received, is currently receiving, or will receive Formula (I) compounds of the present invention.

As used herein, the term "treat" or "treated", with respect to a patient *with* cancer, refers to a patient who has received, is currently receiving, or will receive one or more chemotherapeutic agents and/or Formula (I) compounds of the present invention.

As used herein, "treatment schedule time" or "treatment regimen" means the difference in schedule of administration time, including: (i) the amount of drug administered per day or week; (ii) the amount of drug administered per day or week per m² of body surface area; or (iii) the amount of drug administered per day or week per kg of body weight.

### I. Pharmacology of Taxanes

Taxanes are semi-synthetically derived analogues of naturally occurring compounds derived from plants. In particular, taxanes are derived from the needles and twigs of the European yew *(Taxus baccata),* or the bark of the Pacific yew *(Taxus brevifolia).* The most widely known taxanes at this time are paclitaxel (Taxol^{®}) and docetaxel (Taxotere^{®}), which are widely distributed as antineoplastic agents.

Paclitaxel was discovered in the late 1970s, and was found to be an effective antineoplastic agent with a mechanism of action different from then-existing chemotherapeutic agents. Taxanes are recognized as effective agents in the treatment of many solid tumors which are refractory to other antineoplastic agents.

Paclitaxel has the molecular structure shown below as Formula (A):

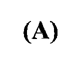

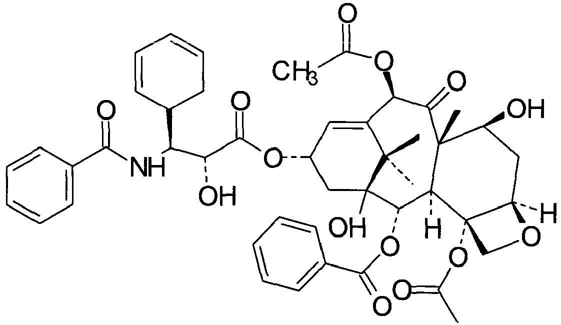

Docetaxel is an analog of Paclitaxel, and has the molecular structure shown below as Formula (B):

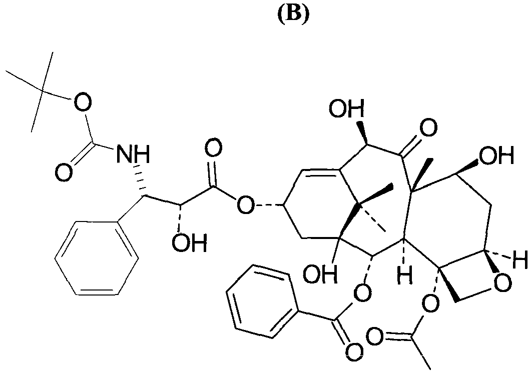

Taxanes exert their biological effects on the cell microtubules and act to promote the polymerization of tubulin, a protein subunit of spindle microtubules. The end result is the inhibition of depolymerization of the microtubules, which causes the formation of stable and nonfunctional microtubules. This disrupts the dynamic equilibrium within the microtubule system, and arrests the cell cycle in the late G₂ and M phases, which inhibits cell replication. Taxanes interfere with the normal function of microtubule growth and arrests the function of microtubules by hyper-stabilizes their structure. This destroys the cell's ability to use its cytoskeleton in a flexible manner.

Taxanes function as an anti-neoplastic agent by binding to the N-terminal 31 amino acid residues of the β-tubulin subunit in tubulin oligomers or polymers, rather than tubulin dimers. Unlike other anti-microtubule agents (e.g., vinca alkaloids) which prevent microtubule assembly, submicromolar concentrations of taxanes function to decrease the lag-time and shift the dynamic equilibrium between tubulin dimers and microtubules *(i.e.,* the hyperpolymerization of tubulin oligomers) toward microtubules assembly and stabilize the newly formed microtubules against depolymerization. The microtubules which are formed are highly stable, thereby inhibiting the dynamic reorganization of the microtubule network. *See, e.g.,* Rowinsky, E.K., et al., Taxol: The prototypic taxane, an important new class of antitumor agents. Semin. Oncol. 19:646 (1992). Tubulin is the "building block" of microtubules, the resulting microtubule/taxane complex does not have the ability to disassemble. Thus, the binding of taxanes inhibit the dynamic reorganization of the microtubule network. This adversely affects cell function because the shortening and lengthening of microtubules (*i.e*., dynamic instability) is necessary for their function as a mechanism to transport other cellular components. For example, during mitosis, microtubules position the chromosomes during their replication and subsequent separation into the two daughter-cell nuclei.

In addition, even at submicromolar concentrations, the taxanes also induce microtubule bundling in cells, as well as the formation of numerous abnormal mitotic asters (which unlike mitotic asters formed under normal physiological conditions, do not require centrioles for enucleation. Thus, the taxanes function to inhibit the proliferation of cells by inducing a sustained mitotic "block" at the metaphase-anaphase boundary at a much lower concentration than that required to increase microtubule polymer mass and microtubule bundle formation. *See, e.g.,* Rao, S., et al., Direct photoaffinity labeling of tubulin with taxol. J. Natl. Cancer Inst. 84:785 (1992). It should be noted that many of the deleterious physiological side-effects caused by the taxanes are caused by the sustained mitotic "block" at the metaphase-anaphase boundary in normal *(i.e.,* non-neoplastic cells).

In addition to stabilizing microtubules, the taxane, paclitaxel, may act as a "molecular sponge" by sequestering free tubulin, thus effectively depleting the cells supply of tubulin monomers and/or dimers. This activity may trigger the aforementioned apoptosis. One common characteristic of most cancer cells is their rapid rate of cell division. In order to accommodate this, the cytoskeleton of the cancer cell undergoes extensive restructuring. Paclitaxel is an effective treatment for aggressive cancers because it adversely affects the process of cell division by preventing this restructuring. Although non-cancerous cells are also adversely affected, the rapid division rate of cancer cells make them far more susceptible to paclitaxel treatment.

Further research has also indicated that paclitaxel, induces programmed cell death (apoptosis) in cancer cells by binding to an apoptosis stopping protein called B-cell leukemia 2 (Bcl-2), thus arresting its function.

The molecular structure of the taxanes are complex alkaloid esters consisting of a taxane system linked to a four-member oxetan ring at positions C-4 and C-5. The taxane rings of both paclitaxel and docetaxel, but not 10-deacetylbaccatin III, are linked to an ester at the C-13 position. Experimental and clinical studies have demonstrated that analogs lacking the aforementioned linkage have very little activity against mammalian tubulin. Moreover, the moieties at C-2' and C-3' are critical with respect to its full biological activity, specifically, for the anti-microtubule hyperpolymerization effect of taxane. The C-2' -OH is of paramount importance for the activity of taxol and the Formula (I) compounds of the present invention, and while the C-2' -OH of taxol can be "substituted" by a sufficiently strong nucleophile (*see*, PCT/US98/21814; page 62, line 8-27) the biological activity would be greatly diminished. *See, e.g.,* Lataste, H., et al., Relationship between the structures of Taxol and baccatine III derivatives and their in vitro action of the disassembly of mammalian brain. Proc. Natl. Acad. Sci. 81:4090 (1984). For example, it has been demonstrated that the substitution of an acetyl group at the C-2' position markedly reduces taxane activity. *See, e.g.,* Gueritte-Voegelein, F., et al., Relationships between the structures of taxol analogues and their antimitotic activity. J. Med. Chem. 34:992 (1991).

Taxanes are toxic compounds having a low therapeutic index which have been shown to cause a number of different toxic effects in patients. The most well-known and severe adverse effects of taxanes are neurotoxicity and hematologic toxicity, particularly anemia and severe neutropenia/thrombocytopenia. Additionally, taxanes also cause hypersensitivity reactions in a large percentage of patients; gastrointestinal effects (*e.g*., nausea, diarrhea and vomiting); alopecia; anemia; and various other deleterious physiological effects, even at the recommended dosages. The Taxane medicaments disclosed in the present invention include, in a non-limiting manner, docetaxel or paclitaxel (including the commercially-available paclitaxel derivatives Taxol^{®} and Abraxane^{®}), polyglutamylated forms of paclitaxel (e.g., Xyotax^{®}), liposomal paclitaxel (e.g., Tocosol^{®}), and analogs and derivatives thereof.

### II. Pharmacology of Platinum Compounds

The anti-neoplastic drug cisplatin (*cis*-diamminedichloroplatinum or "CDDP"), and related platinum based drugs including carboplatin and oxaliplatin, are widely used in the treatment of a variety of malignancies including, but not limited to, cancers of the ovary, lung, colon, bladder, germ cell tumors and head and neck. Platinum complexes are reported to act, in part, by aquation *(i.e.,* to form reactive aqua species), some of which may predominate intracellularly, and subsequently form DNA intra-strand coordination chelation cross-links with purine bases, thereby cross-linking DNA. The currently accepted paradigm with respect to cisplatin's mechanism of action is that the drug induces its cytotoxic properties by forming a reactive monoaquo species that reacts with the N7 nitrogen contained within the imidazole components of guanine and adenosine found in nuclear DNA to form intrastrand platinum-DNA adducts. However, the exact mechanism of action of cisplatin is not completely understood and remains a subject of research interest within the scientific community. Thus, this mechanism is believed to work predominantly through *intra-strand* cross-links, and less commonly, through *inter-strand* cross-links, thereby disrupting the DNA structure and function, which is cytotoxic to cancer cells. Platinum-resistant cancer cells are resilient to the cytotoxic actions of these agents. Certain cancers exhibit intrinsic *de novo* natural resistance to the killing effects of platinum agents and undergo no apoptosis, necrosis or regression following initial platinum compound treatment. In contrast, other types of cancers exhibit cytotoxic sensitivity to platinum drugs, as evidenced by tumor regression following initial treatment, but subsequently develop an increasing level of platinum resistance, which is manifested as a reduced responsiveness and/or tumor growth following treatment with the platinum drug *(i.e.,* "acquired resistance"). Accordingly, new platinum agents are continually being sought which will effectively kill tumor cells, but that are also insensitive or less susceptible to tumor-mediated drug resistance mechanisms that are observed with other platinum agents.

The reaction for cisplatin hydrolysis is illustrated below in Scheme I:

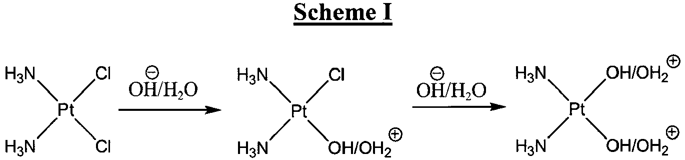

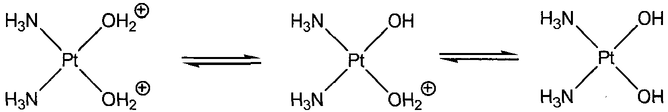

In neutral pH *(i.e.,* pH 7), deionized water, cisplatin hydrolyze to monoaqua/monohydroxy platinum complexes, which is less likely to further hydrolyze to diaqua complexes. However, cisplatin can readily form monoaqua and diaqua complexes by precipitation of chloro ligand with inorganic salts *(e.g.,* silver nitrate, and the like). Also, the chloro ligands can be replaced by existing nucleophile (e.g., nitrogen and sulfur electron donors, etc.) without undergoing aquation intermediates.

Cisplatin is relatively stable in human plasma, where a high concentration of chloride prevents aquation of cisplatin. However, once cisplatin enters a tumor cell, where a much lower concentration of chloride exists, one or both of the chloro ligands of cisplatin is displaced by water to form an aqua-active intermediate form (as shown above), which in turn can react rapidly with DNA purines *(i.e.,* Adenine and Guanine) to form stable platinum-purine-DNA adducts.

Cisplatin enters the cell through both passive diffusion and active transport. The pharmacological behavior of cisplatin is in part determined by hydrolysis reactions that occur once cisplatin is inside the cell where the chloride concentration is essentially zero. In this intracellular *milieu,* one chlorine ligand is replaced by a water molecule to yield an aquated version of cisplatin. The aquated platinum can then react with a variety of intracellular nucleophiles. Cisplatin binds to RNA more extensively than to DNA and to DNA more extensively than to protein; however, all of these reactions are thought to occur intracellularly. Thus, upon administration, a chloride ligand undergoes slow displacement with water (an aqua ligand) molecules, in a process termed aquation. The aqua ligand in the resulting [PtCl(HiO)(NH₃)₂]⁺ is easily displaced, allowing cisplatin to coordinate a basic site in DNA. Subsequently, the platinum cross-links two bases via displacement of the other chloride ligand. Cisplatin crosslinks DNA in several different ways, interfering with cell division by mitosis. The damaged DNA elicits various DNA repair mechanisms, which in turn activate apoptosis when repair proves impossible. Most notable among the DNA changes are the 1,2-intrastrand cross-links with purine bases. These include 1,2-intrastrand d(GpG) adducts which form nearly 90% of the adducts and the less common 1,2-intrastrand d(ApG) adducts. 1,3-intrastrand d(GpXpG) adducts may also occur, but are readily excised by the nucleotide excision repair (NER) mechanism. Other adducts include inter-strand crosslinks and nonfunctional adducts that have been postulated to contribute to cisplatin's activity. In some cases, replicative bypass of the platinum 1, 2-d(GpG) crosslink can occur allowing the cell to faithfully replicate its DNA in the presence of the platinum cross link, but often if this 1,2-intrastrand d(GpG) crosslink is not repaired, it interferes with DNA replication ultimately resulting in apoptosis.

The formation of cisplatin-DNA adducts that interfere with DNA replication is illustrated in Scheme II:

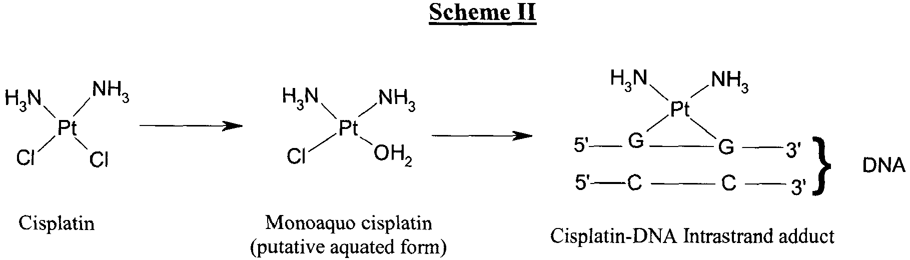

Interaction with cellular proteins, particularly High Mobility Group (HMG) chromosomal domain proteins (which are involved with transcription, replication, recombination, and DNA repair), has also been advanced as a mechanism of interfering with mitosis, although this is probably not its primary method of action. It should also be noted that although cisplatin is frequently designated as an alkylating agent, it has no alkyl group and cannot carry out alkylating reactions. Accordingly, it is more accurately classified as an alkylating-like agent.

Bu way of non-limiting example, the platinum compounds of the present invention include all compounds, compositions, and formulations which containing a platinum ligand in the structure of the molecule. The valence of the platinum ligand contained therein may be platinum II or platinum IV. The platinum medicaments of the present invention include, in a non-limiting manner, cisplatin, oxaliplatin, carboplatin, satraplatin, and analogs and derivatives thereof.

### III. Pharmacology of Formula (I) Compounds

The Formula (I) compounds, most notably for purposes of the present invention, dimesna (disodium-2,2'-dithiobis ethane sulfonate; Tavocept™) and the metabolite of dimesna, mesna (sodium-2-mercaptoethane sulfonate), act to selectively reduce the toxicity of certain antineoplastic agents *in vivo.* Mesna is utilized to reduce the acrolein related uroepithelial cell toxicity of ifosfamide and cyclophosphamide, and is currently approved for such usage in the United States and abroad.

Dimesna is the physiological auto-oxidation dimer of mesna. Mesna (I) and dimesna (II) have the following molecular structures:

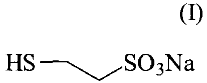

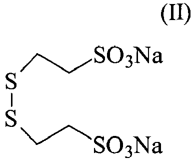

The pharmaceutical chemistry of the compounds indicates that the terminal sulfhydryl group of mesna (and to a lesser extent the disulfide linkage in dimesna) acts as a substitution group for the terminal hydroxy- or aquo- moiety in the active metabolites of platinum complexes. Dimesna, unlike mesna, requires a metabolic activation, such as by glutathione reductase, to exert its biologically efficacious results. Dimesna also exhibits significantly lower toxicity than mesna.

The conversion from the hydroxy- or aquo- moiety to a thioether is favored, particularly under acidic conditions, and results in the formation of a hydrophilic compound of much lower toxicity, one which is rapidly eliminated from the body.

Since blood plasma is slightly alkaline (pH ∼7.3), the more stable disulfide form is the favored species, and does not readily react with the nucleophilic terminal chlorine in cisplatin or the cyclobutane dicarboxylato moiety of carboplatin. This allows the drug to perform its intended cytotoxic action on the targeted cancer cells. Postulated and hypothetical mechanisms of action for the platinum complexes are discussed throughout the recent literature.

The compositions used in the present invention comprise a therapeutically effective amount of a Formula (I) compound, namely 2,2'-dithio-bis-ethane sulfonate or a pharmaceutically-acceptable salt thereof. Compositions of Formula (I), and their synthesis are described in published U.S. Patent Application No. 2005/0256055. It should be noted that *all* of the aforementioned chemical entities in the previous three (3) sentences are included in the terms "Formula (I) compounds" and "Formula (I) compositions" as utilized herein, unless otherwise specifically stated, including the disodium salt of 2,2'-dithio-bis-ethane sulfonate (referred to in the literature as dimesna, Tavocept™, and BNP7787) and the metabolite of disodium 2,2'-dithio-bis-ethane sulfonate, known as 2-mercapto ethane sulfonate sodium (referred to in the literature as mesna).

The putative mechanisms of the Formula (I) compositions of the present invention which function in the potentiation of the anti-cancer activity of chemotherapeutic agents may involve one or more of several novel pharmacological and physiological factors, including but not limited to, a prevention, compromise, and/or reduction in the normal increase, responsiveness, or in the concentration and/or tumor protective metabolism of glutathione/cysteine and other physiological cellular thiols; these antioxidants and enzymes are increased in concentration and/or activity, respectively, in response to the induction of intracellular oxidative stress which may be caused by exposure to cytotoxic chemotherapeutic agents in tumor cells. Additional information regarding certain mechanisms which may be involved in Formula (I) compounds is disclosed in United States Patent Application Serial No. 11/724,933, filed March 16, 2007.

Additionally, disclosure is provided herein which provides evidence that Formula (I) compounds of the present invention also play a role in: (i) increasing patient survival time in cancer patients receiving chemotherapy; (ii) maintaining or stimulating hematological function in patients in need thereof, including those patients suffering from cancer; (iii) maintaining or stimulating erythropoietin function or synthesis in patients in need thereof, including those patients suffering from cancer; (iv) mitigating or preventing anemia in patients in need thereof, including those patients suffering from cancer; (v) maintaining or stimulating pluripotent, multipotent, and unipotent normal stem cell function or synthesis in patients in need thereof, including those patients suffering from cancer; (vi) promoting the arrest or retardation of tumor progression in those cancer patients receiving chemotherapy; and (vii) increasing patient survival and/or delaying tumor progression while maintaining or improving the quality of life in a cancer patient receiving chemotherapy.

### IV Pharmacology of Erythropoietin and the Process of Erythropoiesis

Erythropoiesis is the process by which red blood cells (erythrocytes) are produced. In the early fetus, erythropoiesis takes place in the mesodermal cells of the yolk sac. By the third or fourth month of fetal development, erythropoiesis moves to the spleen and liver. In human adults, erythropoiesis generally occurs within the bone marrow. The long bones of the arm (tibia) and leg (femur) cease to be important sites of hematopoiesis by approximately age 25; with the vertebrae, sternum, pelvis, and cranial bones continuing to produce red blood cells throughout life. However, it should be noted that in humans with certain diseases and in some animals, erythropoiesis also occurs outside the bone marrow, within the spleen or liver. This is termed extramedullary erythropoiesis.

In the process of red blood cell maturation, a cell undergoes a series of differentiations. The following stages of development all occur within the bone marrow: (i) pluripotent hematopoietic stem cell; (ii) multipotent stem cell; (iii) unipotent stem cell; (iv) pronormoblast; (v) basophilic normoblast/early normoblast; (vi) polychrmatophilic normoblast/intermediate normoblast; (vii) orthochromic normoblast/late normoblast; and (viii) reticulocyte. Following these stages, the cell is released from the bone marrow, and ultimately becomes an "erythrocyte" or mature red blood cell circulating in the peripheral blood. These stages correspond to specific histological appearances of the cell when stained with Wright's stain and examined via light microscopy, but they also correspond to numerous other intrinsic biochemical and physiological changes. For example, in the process of maturation, a basophilic pronormoblast is converted from a cell with a large nucleus and a volume of 900 µm³ to an enucleated disc with a volume of 95 µm³. By the reticulocyte stage, the cell has extruded its nucleus, but is still capable of producing hemoglobin.

A feedback loop involving the cytokine glycoprotein hormone erythropoietin (discussed below) helps regulate the process of erythropoiesis so that, in non-disease states, the production of red blood cells is equal to the destruction of red blood cells and the red blood cell number is sufficient to sustain adequate tissue oxygen levels but not so high as to cause blood thickening or "sludging", thrombosis, and/or stroke. Erythropoietin is produced in the kidney and liver in response to low oxygen levels. In addition, erythropoietin is bound by circulating red blood cells; low circulating numbers lead to a relatively high level of unbound erythropoietin, which stimulates production in the bone marrow.

Recent studies have also shown that the peptide hormone hepcidin may also play a role in the regulation of hemoglobin production, and thus effect erythropoiesis. Hepcidin, produced by the liver, controls iron absorption in the gastrointestinal tract and iron release from reticuloendothelial tissue. Iron must be released from macrophages in the bone marrow to be incorporated into the heme group of hemoglobin in erythrocytes.

There are colony forming units (e.g., including the granulocyte monocyte colony forming units) that cells follow during their formation. These cells are referred to as the committed cells. For example, the loss of function of the erythropoietin receptor or JAK2 in mice cells causes failure in erythropoiesis, so production of red blood cells in embryos and growth is disrupted. Similarly, the lack of feedback inhibition, such as SOCS (Suppressors of Cytokine Signaling) proteins in the system, have been shown to cause giantism in mice.

Erythropoietin (EPO) is a cytokine glycoprotein hormone that is a cytokine for erythrocyte (red blood cell) precursors in the bone marrow which regulates the process of red blood cell production (erythropoiesis). Cytokines are a group of proteins and peptides that function as signaling compounds produced by cells to communicate with one another. They act via cell-surface cytokine receptors. The cytokine family consists mainly of smaller water-soluble proteins and glycoproteins *(i.e.,* proteins with an added sugar chain(s)) with a mass of between 8 and 30 kDa. They act like hormones and neurotransmitters but whereas hormones are released from specific organs into the blood and neurotransmitters are produced by neurons, cytokines are released by many types of cells. Due to their central role in the immune system, cytokines are involved in a variety of immunological, inflammatory, and infectious diseases. When the immune system is fighting pathogens, cytokines signal immune cells such as T-cells and macrophages to travel to the site of infection. In addition, cytokines activate those cells, stimulating them to produce more cytokines. However, not all their functions are limited to the immune system, as they are also involved in several developmental processes during embryogenesis. Cytokines are produced by a wide variety of cell types (both hemopoietic and non-hemopoietic), and can have effects on both nearby cells or throughout the organism. Sometimes these effects are strongly dependent on the presence of other chemicals and cytokines. Cytokines may be synthesized and administered exogenously. However, such molecules can, at a latter stage be detected, since they differ slightly from the endogenous ones in, *e.g*., features of post-translational modification.

EPO is produced mainly by peritubular fibroblasts of the renal cortex. Regulation is believed to rely on a feed-back mechanism measuring blood oxygenation. Constitutively synthesized transcription factors for EPO, known as hypoxia inducible factors (HIFs), are hydroxylized and proteosomally-digested in the presence of oxygen. *See, e.g.,* Jelkmann, W. Erythropoietin after a century of research: younger than ever. Eur. J. Haematol. 78 (3):183-205 (2007). Hypoxia-inducible factors (HIFs) are transcription factors that respond to changes in available oxygen in the cellular environment, in specific, to decreases in oxygen, or hypoxia. Most, if not all, oxygen-breathing species express the highly-conserved transcriptional complex HIF-1, which is a heterodimer composed of an α- and a β-subunit, the latter being a constitutively-expressed aryl hydrocarbon receptor nuclear translocator (ARNT).

HIF-1 belongs to the PER-ARNT-SIM (PAS) subfamily of the basic helix-loop-helix (bHLH) family of transcription factors. The α-subunit of HIF-1 is a target for propyl hydroxylation by HIF prolyl-hydroxylase, which makes HIF-1α a target for degradation by the E3 ubiquitin ligase complex, leading to quick degradation by the proteosome. This occurs only in normoxic conditions. In hypoxic conditions, HIF prolyl-hydroxylase is inhibited, since it utilizes oxygen as a co-substrate.

Hypoxia also results in a buildup of succinate, due to inhibition of the electron transport chain in the mitochondria. The buildup of succinate further inhibits HIF prolyl-hydroxylase action, since it is an end-product of HIF hydoxylation. In a similar manner, inhibition of electron transfer in the succinate dehydrogenase complex due to mutations in the SDHB or SDHD genes can cause a build-up of succinate that inhibits HIF prolyl-hydroxylase, stabilizing HIF-1 a. This is termed pseudohypoxia.

HIF-1, when stabilized by hypoxic conditions, upregulates several genes to promote survival in low-oxygen conditions. These include glycolysis enzymes, which allow ATP synthesis in an oxygen-independent manner, and vascular endothelial growth factor (VEGF), which promotes angiogenesis. HIF-1 acts by binding to HIF-responsive elements (HREs) in promoters that contain the sequence NCGTG. In general, HIFs are vital to development. In mammals, deletion of the HIF-1 genes results in perinatal death. HIF-1 has been shown to be vital to chondrocyte survival, allowing the cells to adapt to low-oxygen conditions within the growth plates of bones.

Erythropoietin is available as a therapeutic agent produced by recombinant DNA technology in mammalian cell culture. It is used in treating anemia resulting from chronic kidney disease, from the treatment of cancer (e.g., from chemotherapy and radiation) and from other critical illnesses (e.g., heart failure).

In should be noted that there have been a number of recent warnings released by both pharmaceutical manufacturers and the United States Food and Drug Administration (FDA) concerning the safety of EPO use in anemic cancer patients. Initially, a manufacturer of erythropoiesis-stimulating agents (ESAs), disseminated a "Dear Doctor" letter in 2007, that highlighted results from a recent clinical trial which examined cancer-associated anemia, and warned doctors to consider use in that off-label indication with caution. An ESA manufacturer also advised the FDA regarding the results of three (3) clinical trials: the DAHANCA 10; PREPARE, and GOG-191 clinical trials. For example, DAHANCA refers to a series of studies, entitled "Danish Head and Neck Cancer Studies" the most recent of which is "DAHANCA 10". *See. e.g.,* Eriksen, J. and Overgaard, J., Lack of prognostic and predictive value of CA IX in radiotherapy of squamous cell carcinoma of the head and neck with known modifiable hypoxia: An evaluation of the DAHANCA 5 study. Radiotherap. Oncol. 83(3):383-388 (2007). In this study, the DAHANCA 10 data monitoring committee found that three year loco-regional control of various types of head and neck cancers in subjects treated with an ESA was significantly worse than for those not receiving an ESA (p=0.01). In response to these advisories, the FDA subsequently released a Public Health Advisory and a clinical alert for physicians, regarding the use of ESAs. The advisory recommended caution in using these agents in cancer patients receiving chemotherapy or off chemotherapy, and indicated a lack of clinical evidence to support improvements in quality of life or transfusion requirements in these settings. In addition, ESA manufacturers have agreed to new Black Box Warnings about the safety of these drugs. It should be noted that, additional information regarding various ESAs may be obtained from the Food and Drug Administration (FDA) or the specific ESA manufactures themselves.

A related cytokine, colony-stimulating factors (CSF), are secreted glycoproteins which bind to receptor proteins on the surfaces of hematopoietic stem cells and thereby activate intracellular signaling pathways which can cause the cells to proliferate and differentiate into a specific kind of blood cell (typically white blood cells). Hematopoietic stem cells (HSC) are stem cells *(i.e.,* cells retain the ability to renew themselves through mitotic cell division and can differentiate into a diverse range of specialized cell types) that give rise to all the blood cell types including myeloid (e.g., monocytes, macrophages, neutrophils, basophils, eosinophils, erythrocytes, megakaryocytes/platelets, dendritic cells, and the like) and lymphoid lineages (e.g., T-cells, B-cells, NK-cells, and the like). The definition of hematopoietic stem cells has undergone considerable revision in the last two decades. The hematopoietic tissue contains cells with long-term and short-term regeneration capacities and committed multipotent, oligopotent, and unipotent progenitors. Recently, long-term transplantation experiments point toward a clonal diversity model of hematopoietic stem cells. Here, the HSC compartment consists of a fixed number of different types of HSC, each with epigenetically-preprogrammed behavior. This contradicts older models of HSC behavior, which postulated a single type of HSC that can be continuously molded into different subtypes of HSCs. For example, HSCs constitute 1:10.000 of cells in myeloid tissue.

Colony-stimulating factors may be synthesized and administered exogenously. However, such molecules can at a latter stage be detected, since they differ slightly from endogenous ones in *e.g.*, post-translational modification. The name "colony-stimulating factors" comes from the method by which they were discovered. Hemopoietic stem cells were cultured on a so-called semi solid matrix which prevents cells from moving around, so that if a single cell starts proliferating, all of the cells derived from it will remain clustered around the spot in the matrix where the first cell was originally located, and these are referred to as "colonies." It was therefore possible to add various substances to cultures of hemopoietic stem cells and then examine which kinds of colonies (if any) were "stimulated" by them. The substance which was found to stimulate formation of colonies of macrophages, for instance, was called macrophage colony-stimulating factor, and so on. The colony-stimulating factors are soluble, in contrast to other, membrane-bound substances of the hematopoietic microenvironment. This is sometimes used as the definition of CSF. They transduce by paracrine, endocrine, or autocrine signaling.

Colony-stimulating factors include: macrophage colony-stimulating factor; granulocyte-macrophage colony-stimulating factor; and granulocyte colony-stimulating factor. Macrophage colony-stimulating factor (M-CSF or CSF-1), is a secreted cytokine which influences hematopoietic stem cells to differentiate into macrophages or other related cell types. M-CSF binds to the macrophage colony-stimulating factor receptor. It may also be involved in development of the placenta.

Granulocyte-macrophage colony-stimulating factor (GM-CSF or CSF-2), is a protein secreted by macrophages, T-cells, mast cells, endothelial cells, and fibroblasts. GM-CSF is a cytokine that functions as a white blood cell growth factor. GM-CSF stimulates stem cells to produce granulocytes (e.g., neutrophils, eosinophils, and basophils) and monocytes. Monocytes exit the circulation and migrate into tissue, whereupon they mature into macrophages. It is thus part of the immune/inflammatory cascade, by which activation of a small number of macrophages can rapidly lead to an increase in their numbers, a process crucial for fighting infection. The active form of the protein is found extracellularly as a homodimer.

Granulocyte Colony-Stimulating Factor (G-CSF or CSF-3), is a colony-stimulating factor hormone. It is a glycoprotein, growth factor, or cytokine produced by a number of different tissues to stimulate the bone marrow to produce granulocytes and stem cells. G-CSF then stimulates the bone marrow to pulse them out of the marrow into the blood. It also stimulates the survival, proliferation, differentiation, and function of neutrophil precursors and mature neutrophils. G-CSF is produced by endothelium, macrophages, and a number of other immune cells. The natural human glycoprotein exists in two forms, a 174- and 180-amino acids-long protein of molecular weight 19,600 grams per mole. The more-abundant and more-active 174-amino acid form has been used in the development of pharmaceutical products by recombinant DNA (rDNA) technology. The G-CSF receptor is present on precursor cells in the bone marrow, and, in response to stimulation by G-CSF, initiates proliferation and differentiation into mature granulocytes. Promegapoietin is a recombinant drug which is given during chemotherapy to increase blood cell regeneration. It is a colony-stimulating factor that stimulates megakaryocyte production. It functions by stimulating ligands for interleukin-3 and c-Mpl.

Chemotherapeutic agents may be prepared and administered to subjects using methods known within the art. For example, paclitaxel may be prepared using methods described in U.S. Patent Nos. 5,641,803, 6,506,405, and 6,753,006 and is administered as known in the art *(see, e.g.,* U.S. Patent Nos. 5,641,803, 6,506,405, and 6,753,006). Paclitaxel may be prepared for administration in a dose in the range of 50 mg/m² to 275 mg/m². Preferred doses include 160 mg/m² to 190 mg/m². The most preferred dose is about 175 mg/m².

Docetaxel may be prepared using methods described in U.S. Patent No. 4,814,470 and is administered as known in the art *(see, e.g.,* U.S. Patent Nos., 4,814,470, 5,438,072, 5,698,582, and 5,714,512). Docetaxel may be prepared for administration in a dose in the range of 30 mg/m² to 100 mg/m². Preferred doses include about 55 mg/m², about 60 mg/m², about 75 mg/m², and about 100 mg/m².

Cisplatin may be prepared using methods described in U.S. Patent Nos. 4,302,446, 4,322,391, 4,310,515, and 4,915,956 and is administered as known in the art *(see, e.g.,* U.S. Patent Nos. 4,177,263, 4,310,515, 4,451,447). Cisplatin may be prepared for administration in a dose in the range of 30 mg/m² to 120 mg/m² in a single dose. Preferred doses range from 60 mg/m² to 100 mg/m². The most preferred doses range from 75 mg/m² to 85 mg/m².

Carboplatin may be prepared using methods described in U.S. Patent No. 4,657,927 and is administered as known in the art *(see, e.g.,* U.S. Patent No. 4,657,927). Carboplatin may be prepared for administration in a dose in the range of 20 mg/kg and 200 mg/kg. Preferred doses include about 300 mg/m² and about 360 mg/m². Other dosing may be calculated using a formula according to the manufacturer's instructions.

Oxaliplatin may be prepared using methods described in U.S. Patent Nos. 5,290,961, 5,420,319, 5,338,874 and is administered as known in the art (*see*, *e.g.,* U.S. Patent No. 5,716,988). Oxaliplatin may be prepared for administration in a dose in the range of 50 mg/m² and 200 mg/m². Preferred doses include about 85 mg/m² and about 130 mg/m².

The compositions of Formula (I) include 2,2'-dithio-bis-ethane sulfonate and a pharmaceutically-acceptable salt thereof. Pharmaceutically-acceptable salts used in the present invention include, but are not limited to: (i) a monosodium salt; (ii) a sodium potassium salt; (iii) a dipotassium salt; (iv) a calcium salt; (v) a magnesium salt; (vi) a manganese salt; (vii) an ammonium salt; (viii) a monopotassium salt; and (ix) most preferably, disodium. It should be noted that mono- and di-potassium salts are only administered to a subject if the total dose of potassium administered at any given point in time is not greater than 100 Meq., the subject is not hyperkalemic, and/or the subject does not have a condition that would predispose the subject to hyperkalemia (e.g., renal failure).

By way of non-limiting example, disodium 2,2'-dithio-bis-ethane sulfonate (also referred to in the literature as dimesna, Tavocept™, and BNP7787) is a known compound and can be manufactured by methods known in the art. *See, e.g.,* J. Org. Chem. 26:1330-1331 (1961); J. Org. Chem. 59:8239 (1994). In addition, various salts of 2,2'-dithio-bis-ethane sulfonate, as well as other dithioethers may also be synthesized as outlined in U.S. Patent No. 5,808,160, U.S. Patent No. 6,160,167 and U.S. Patent No. 6,504,049. Compounds of Formula (I) may be manufactured as described in Published U.S. Patent Application 2005/0256055

Preferred doses of the Formula (I) compounds of the present invention range from 14 g/m² to 22 g/m², with a most preferred dose of 18.4 g/m².

A better understanding of the invention will be gained by reference to the following section of Specific Examples and Experimental Results. The following examples are illustrative and are not intended to limit the invention or the claims in any way.

### Specific Examples and Experimental Results

### I. Japan Phase III Clinical Trial

### A. Summary of the objectives and methods of the Japan phase III clinical trial

Data was recently unblinded from a multicenter double-blind randomized placebo-controlled Phase III clinical trial of the Formula (I) compound Tavocept™ (also known as BNP7787, disodium 2,2'-dithio-bis-ethane sulfonate, and dimesna) conducted in Japan (hereinafter the "Japan Phase III Clinical Trial") and involving patients with advanced non-small cell lung carcinoma (NSCLC) who received the chemotherapeutic drugs paclitaxel and cisplatin.

The primary objective of the Japan Phase III Clinical Trial was to show that the Formula (I) compound, Tavocept™, prevents and/or reduces peripheral neuropathy induced by paclitaxel + cisplatin combination therapy in patients with non-small cell lung carcinoma (NSCLC).

Patients admitted into the trial included those patients without previous treatment (excluding surgical treatment, administration of Picibanil into the serous membrane, irradiation of 30% or less hematopoietic bone, or oral chemotherapeutic agents within 3 months of entry in the trial).

The Japan Phase III Clinical Trial was conducted as a double-blind study because peripheral neuropathy is diagnosed based on subjective symptoms evaluated through clinical interviews, lab tests, and the like. Accordingly, evaluations by both physicians and patients are highly important. The present trial was designed to show that Tavocept™ prevents and/or reduces peripheral neuropathy induced by paclitaxel and cisplatin in NSCLC patients. A placebo was used as control since there is no established therapy or drug for preventing peripheral neuropathy. Because the severity of peripheral neuropathy is evaluated based on patients' reports *(i.e.,* subjective symptoms), the Peripheral Neuropathy Questionnaire (PNQ^{©}) was used in primary evaluation. CIPN-20 and NCI-CTC were used in secondary evaluation. The incidence and severity of adverse reactions, time to their onset, etc. and the like, were compared between patients treated with Tavocept™ and those given a placebo using the aforementioned methods.

In order to conduct the present trial, Tavocept™ (approximately14-22 g/m², most preferably approximately 18.4 g/m²) or placebo (0.9% NaCl) was administered to non-small cell lung carcinoma (NSCLC) patients receiving chemotherapy with paclitaxel (approximately 160-190 mg/m², most preferably approximately 175 mg/m²) and cisplatin (approximately 60-100, most preferably approximately 80 mg/m²), every 3 weeks (and repeated for a minimum of 2 cycles).

### B. Summary of the results of the Japan phase III clinical trial

The Japan Phase III Clinical Trial data demonstrated medically-important reductions in chemotherapy-induced peripheral neuropathy for patients receiving Tavocept™ and chemotherapy compared to patients receiving chemotherapy and a placebo. In addition, there were concurrent observations in the clinical trial population of medically-important reductions in chemotherapy-induced vomiting/emesis and kidney damage.

The aforementioned clinical trial also provided a number of unexpected physiological results which have, heretofore, been unreported in any previous scientific or clinical studies. Importantly, the Japan Phase III Clinical Trial demonstrated increased survival times for patients with advanced non-small cell lung cancer (NSCLC) receiving Tavocept™ and chemotherapy. A medically-important increase in survival time was also observed in patients with adenocarcinoma receiving Tavocept™ and chemotherapy. In addition, these unexpected and novel results included, but were not limited to, (i) the differentiation of chemotherapy-induced peripheral neuropathy into an entirely new class of peripheral neuropathy, called "intermittent" or "sporadic" peripheral neuropathy; (ii) potentiation of the cytotoxic or apoptotic activities of chemotherapeutic agents in patients with non-small cell lung carcinoma and adenocarcinoma receiving Tavocept™ and chemotherapy; (iii) increasing patient survival and/or delaying tumor progression while maintaining or improving the quality of life in patients with non-small cell lung carcinoma and adenocarcinoma receiving Tavocept™ and chemotherapy; and (iv) the maintenance or stimulation of hematological function (e.g., an increase in hemoglobin, hematocrit, and erythrocyte levels), in patients with non-small cell lung carcinoma and adenocarcinoma receiving Tavocept™ and chemotherapy.

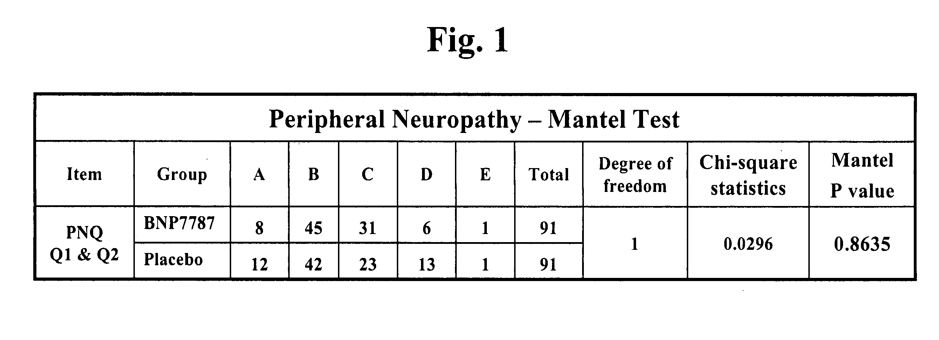

Fig. 1 illustrates, in tabular form, the Primary Endpoint *(i.e.,* the mitigation or prevention of patient peripheral neuropathy) of the Japan Phase III Clinical Trial supporting the present invention as determined utilizing the Peripheral Neuropathy Questionnaire (PNQ^{©}). Results illustrated in Fig. 1 demonstrate that there was an approximate 50% reduction in severe (Grade D or E) peripheral neuropathy in the patient population with non-small cell lung carcinoma (NSCLC) who were treated with a paclitaxel/Tavocept™/cisplatin regimen in comparison to those NSCLC patients who received a paclitaxel/saline placebo/cisplatin regimen.

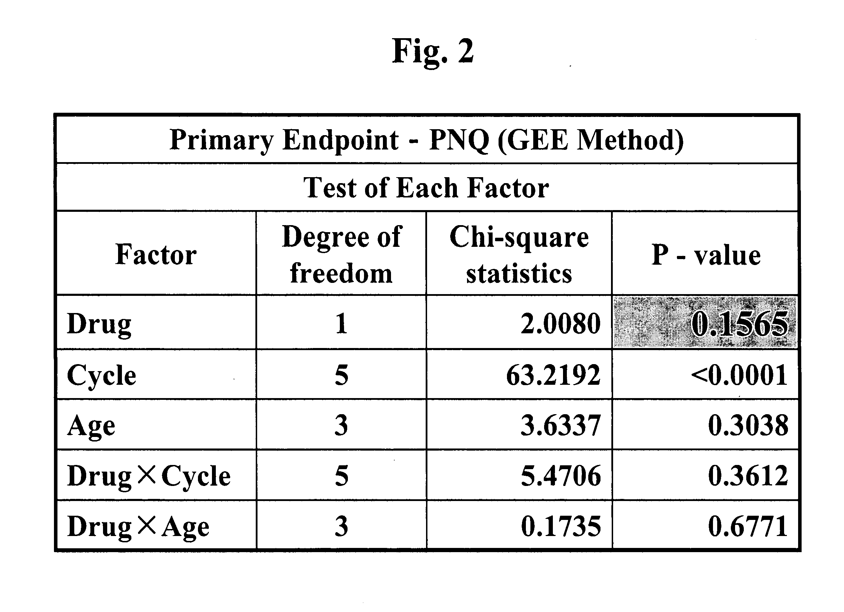

Fig. 2 illustrates, in tabular form, an evaluation of the statistical power observed in the Japan Phase III Clinical Trial with respect to the Primary Endpoint (*i.e.,* the mitigation or prevention of patient peripheral neuropathy), as measured by the Generalized Estimating Equation (GEE) statistical method. The numerical value of 0.1565 in the tabular row designated "Drug" under the tabular column designated "P-Value" in Fig. 2, demonstrates that there is only a 15.65% probability that the reduction in peripheral neuropathy observed for Tavocept™ in the Japan Phase III Clinical Trial is due to random chance alone.

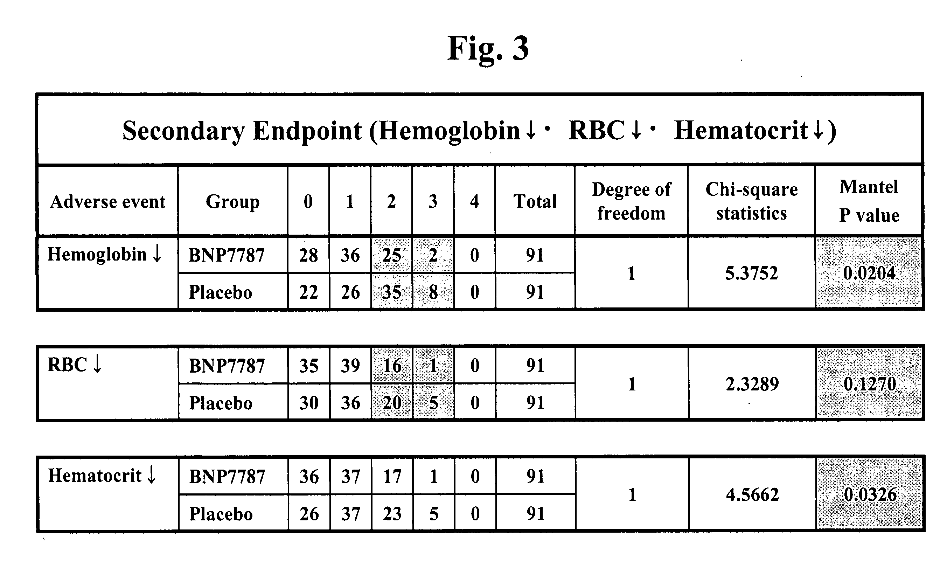

Fig. 3 illustrates, in tabular form, at Secondary Endpoint (*i.e.,* a decrease in patient hemoglobin, erythrocyte, and hematocrit levels) of the Japan Phase III Clinical Trial supporting the present invention, in patients receiving Tavocept™ and chemotherapy. Results illustrated in Fig. 3 demonstrate that only 2, 1, and 1 non-small cell lung carcinoma (NSCLC) patients in the Tavocept™ arm of the study exhibited a Grade 3 (severe) decrease in hemoglobin, red blood cell, and hematocrit levels, respectively, in comparison to 8, 5, and 5 patients in identical categories in the placebo arm of the Japan Phase III Clinical Trial.

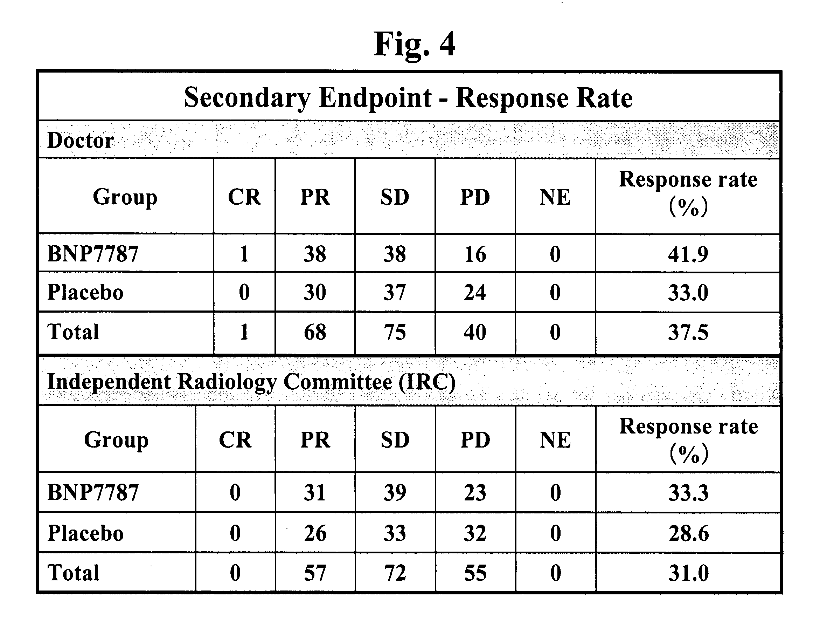

Fig. 4 illustrates, in tabular form, a Secondary Endpoint (*i.e.,* tumor response rate to chemotherapy administration) of the Japan Phase III Clinical Trial supporting the present invention, in patient populations receiving either Tavocept™ or placebo, as measured by the physician or by the Independent Radiological Committee (IRC) criteria. As is shown in the portion of the table designated "Doctor", the Response Rate, as measured by physicians, in the Tavocept™ arm of the Japan Phase III Clinical Trial was 41.9% compared to a 33.0% Response Rate in the placebo arm. As shown in the portion of the table designated "IRC", the response rate as measured by the IRC in the Tavocept™ arm of the Japan Phase III Clinical Trial was 33.3% as compared to a 28.6% response rate in the placebo arm.

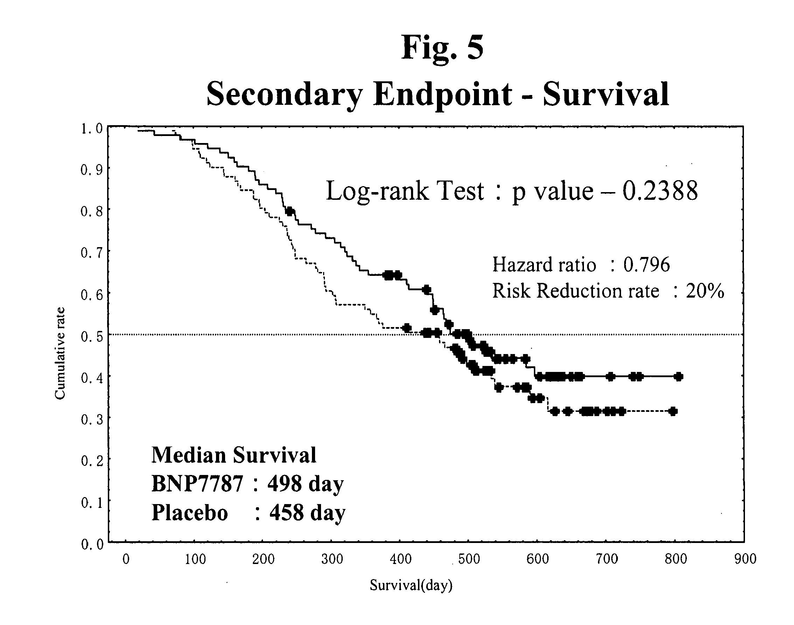

Fig. 5 illustrates, in graphical form, a Secondary Endpoint (*i.e*., patient survival) of the Japan Phase III Clinical Trial supporting the present invention, in patient populations receiving either Tavocept™ or placebo. Results illustrated in Fig. 5 demonstrate an increase in median survival time of up to 40 days in the portion of the patient population with non-small cell lung carcinoma (NSCLC) who were treated with a paclitaxel/Tavocept™/cisplatin regimen in comparison to median survival time for those NSCLC patients who received a paclitaxel/saline placebo/cisplatin regimen.

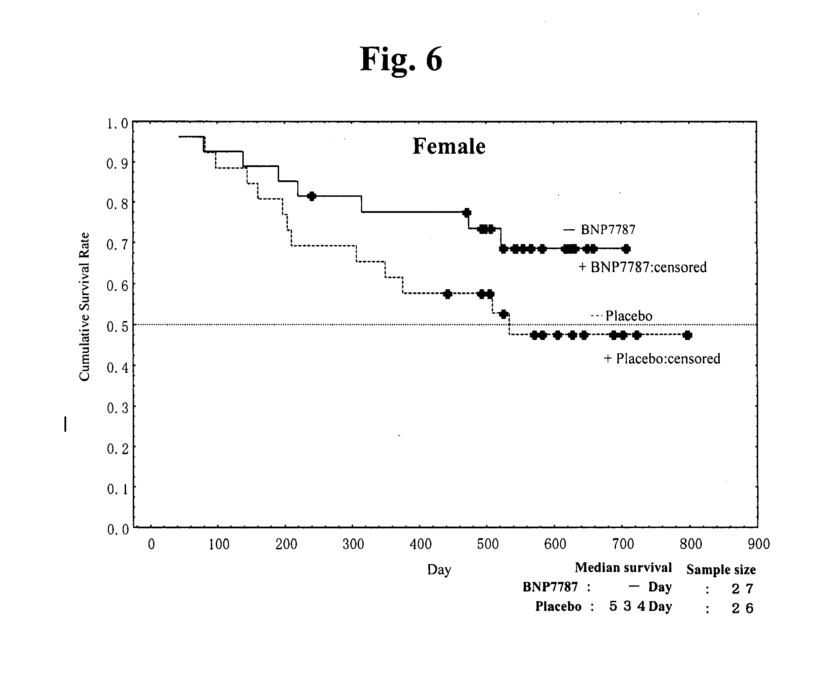

Fig. 6 illustrates, in graphical form, a Secondary Endpoint *(i.e.,* patient survival) of the Japan Phase III Clinical Trial supporting the present invention, in female patient populations receiving either Tavocept™ or placebo. Results in Fig. 6 demonstrate that the portion of the female patient population with non-small cell lung carcinoma (NSCLC) who were treated with a paclitaxel/Tavocept™/cisplatin regimen had a longer survival period in comparison to the female NSCLC patient population who received a paclitaxel/saline placebo/cisplatin regimen.

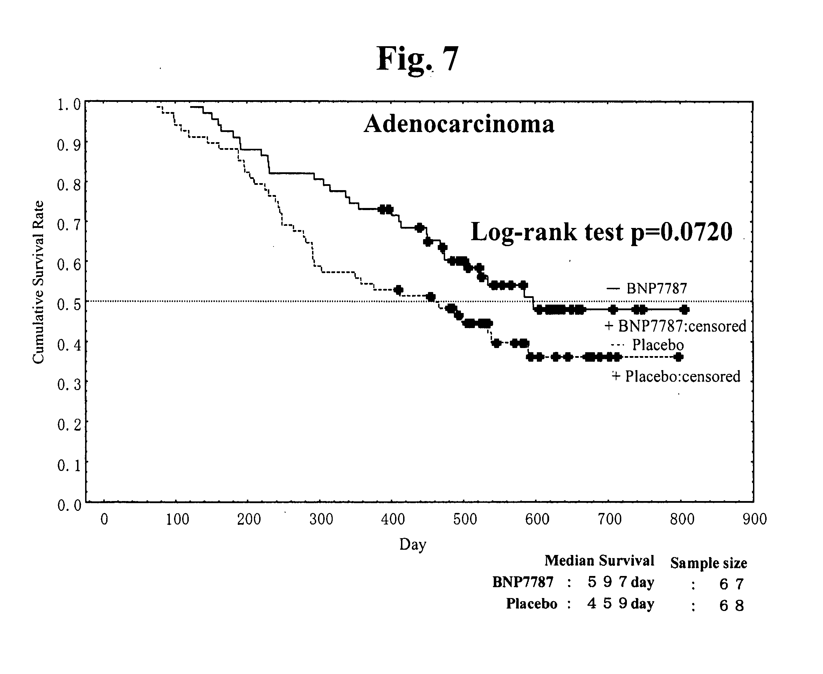

Fig. 7 illustrates, in graphical form, a Secondary Endpoint *(i.e.,* patient survival) of the Japan Phase III Clinical Trial supporting the present invention, in patient populations diagnosed with adenocarcinoma receiving either Tavocept™ or placebo. Results illustrated in Fig. 7 demonstrate an increase in median survival time of up to 138 days in the portion of the patient population with adenocarcinoma who were treated with a paclitaxel/Tavocept™/cisplatin regimen in comparison to the median survival time for those adenocarcinoma patients who received a paclitaxel/saline placebo/cisplatin regimen.

In addition, results from the Japan Phase III Clinical Trial also demonstrated reductions in: (i) fatigue (p = 0.0163); (ii) nausea/vomiting (p = 0.0240); (iii) anorexia (p = 0.0029).; (iv) diarrhea (p = 0.0859); (v) constipation (p = 0.1114); and (vi) insomnia (p = 0.1108) in the portion of the patient population with non-small cell lung carcinoma (NSCLC) who were treated with a paclitaxel/Tavocept™/cisplatin regimen in comparison to those NSCLC patients who received a paclitaxel/saline placebo/cisplatin regimen.

The results from the Japan Phase III Clinical Trial described in the instant application represent medically important developments that support surprising new findings for Formula (I) compounds, including potential uses for: (i) increasing patient survival time in cancer patients receiving chemotherapy; (ii) causing cytotoxic or apoptotic potentiation of the anti-cancer activity of chemotherapeutic agents in cancer patients receiving chemotherapy; (iii) maintaining or stimulating hematological function in patients in need thereof, including cancer patients; (iv) maintaining or stimulating erythropoietin function or synthesis in patients in need thereof, including cancer patients; (v) mitigating or preventing anemia in patients in need thereof, including cancer patients; (vi) maintaining or stimulating pluripotent, multipotent, and unipotent normal stem cell function or synthesis in patients in need thereof, including cancer patients; (vii) promoting the arrest or retardation of tumor progression in those cancer patients receiving chemotherapy; and (viii) increasing patient survival and/or delaying tumor progression while maintaining or improving the quality of life in cancer patients receiving chemotherapy.

## Claims

1. 2,2'-Dithio-bis-ethane sulfonate or a pharmaceutically acceptable salt thereof, for use in mitigating or preventing chemotherapy-induced anemia in a patient, wherein the patient suffers from lung cancer or adenocarcinoma and is treated with a taxane medicament and/or cisplatin.

2. 2,2'-Dithio-bis-ethane sulfonate or a pharmaceutically acceptable salt thereof, for use according to Claim 1, wherein said lung cancer is non-small cell lung carcinoma.

3. 2,2'-Dithio-bis-ethane sulfonate or a pharmaceutically acceptable salt thereof, for use according to Claim 1 or 2, wherein said anemia is mitigated or prevented in said patient by:
a) maintaining, protecting, or stimulating erythropoiesis in the bone marrow;
b) increasing synthesis or half-life of erythropoietin;
c) maintaining the normal physiological regulation of the biosynthesis, secretion, and feedback controls of erythropoiesis; and/or
d) decreasing turnover of circulating erythrocytes.

4. 2,2'-Dithio-bis-ethane sulfonate or a pharmaceutically acceptable salt thereof, for use according to any of Claims 1 to 3, wherein said salt is disodium 2,2'-dithio-bis-ethane sulfonate.

5. 2,2'-Dithio-bis-ethane sulfonate or a pharmaceutically acceptable salt thereof, for use according to any of Claims 1 to 4, wherein said patient is treated with a taxane medicament selected from the group consisting of: docetaxel, paclitaxel, polyglutamylated forms of paclitaxel and liposomal paclitaxel.

6. 2,2'-Dithio-bis-ethane sulfonate or a pharmaceutically acceptable salt thereof, for use according to any of Claims 1 to 5, wherein said patient is treated with cisplatin and paclitaxel.

7. 2,2'-Dithio-bis-ethane sulfonate or a pharmaceutically acceptable salt thereof, for use according to Claim 1, wherein said patient is suffering from lung cancer and is to be treated by the administration of paclitaxel, said sulfonate or salt, and cisplatin once every 2-4 weeks, wherein the dose of paclitaxel is to range from 160 mg/m² to 190 mg/m², the dose of the sulfonate or salt is to range from 14 g/m² to 22 g/m², and the dose of cisplatin is to range from 60 mg/m² to 100 mg/m², wherein said administration of paclitaxel, sulfonate or salt, and cisplatin once every 2-4 weeks is to be repeated at least once.

8. 2,2'-Dithio-bis-ethane sulfonate or a pharmaceutically acceptable salt thereof, for use according to Claim 1, wherein said patient is suffering from lung cancer and is to be treated by the administration of paclitaxel, the sulfonate or salt, and cisplatin once every 3 weeks, wherein the dose of paclitaxel is to be approximately 175 mg/m², the dose of the sulfonate or salt is to be approximately 18.4 g/m², and the dose of cisplatin is to range from 75 mg/m² to 85 mg/m², wherein said administration of paclitaxel, sulfonate or salt, and cisplatin once every 3 weeks is to be repeated for 6 cycles.

9. 2,2'-Dithio-bis-ethane sulfonate or a pharmaceutically acceptable salt thereof, for use according to Claim 1, wherein a taxane medicament and cisplatin and the sulfonate or salt are to be administered to a patient with lung cancer or adenocarcinoma, wherein: (a) the dose rate of the taxane medicament and cisplatin are to range from 10-20 mg/m²/day and a dose rate of the sulfonate or salt is to range from 4.1-41.0 g/m² per day; (b) the concentration of the taxane medicament and cisplatin and/or sulfonate or salt is at least 0.01 mg/mL; (c) the infusion time of the taxane medicament and cisplatin and/or sulfonate or salt is from 5 minutes to 24 hours, and can be repeated as needed and tolerated in a given patient; and (d) the schedule of administration of the taxane medicament and cisplatin and/or sulfonate or salt is every 2-8 weeks.

## Patentansprüche

1. 2,2'-Dithio-bis-ethansulfonat oder ein pharmazeutisch akzeptables Salz davon zur Verwendung bei der Linderung oder dem Vermeiden einer chemotherapieinduzierten Anämie in einem Patienten, wobei der Patient an Lungenkrebs oder einem Adenokarzinom leidet und mit einem Taxan-Medikament und/oder Cisplatin behandelt wird.

2. 2,2'-Dithio-bis-ethansulfonat oder ein pharmazeutisch akzeptables Salz davon zur Verwendung nach Anspruch 1, wobei der Lungenkrebs ein nicht-kleinzelliges Lungenkarzinom ist.

3. 2,2'-Dithio-bis-ethansulfonat oder ein pharmazeutisch akzeptables Salz davon zur Verwendung nach Anspruch 1 oder 2, wobei die Anämie in dem Patienten gelindert oder vermieden wird, indem:
a) im Knochenmark Erythropoese aufrechterhalten, geschützt und stimuliert wird;
b) die Synthese oder Halbwertszeit von Erythropoetin erhöht wird;
c) die normale physiologische Regulation der Biosynthese, Sekretion und Rückkopplungsregelungen der Erythropoese aufrechterhalten wird; und/oder
d) der Umsatz von zirkulierenden Erythrozyten vermindert wird.

4. 2,2'-Dithio-bis-ethansulfonat oder ein pharmazeutisch akzeptables Salz davon zur Verwendung nach einem der Ansprüche 1 bis 3, wobei das Salz Dinatrium-2,2'-dithio-bis-ethansulfonat ist.

5. 2,2'-Dithio-bis-ethansulfonat oder ein pharmazeutisch akzeptables Salz davon zur Verwendung nach einem der Ansprüche 1 bis 4, wobei der Patient mit einem Taxan-Medikament behandelt wird, das aus der Gruppe, bestehend aus Docetaxel, Paclitaxel, polyglutamylierte Formen von Paclitaxel und liposomalem Paclitaxel, ausgewählt ist.

6. 2,2'-Dithio-bis-ethansulfonat oder ein pharmazeutisch akzeptables Salz davon zur Verwendung nach einem der Ansprüche 1 bis 5, wobei der Patient mit Cisplatin und Paclitaxel behandelt wird.

7. 2,2'-Dithio-bis-ethansulfonat oder ein pharmazeutisch akzeptables Salz davon zur Verwendung nach Anspruch 1, wobei der Patient an Lungenkrebs leidet und durch die Verabreichung von Paclitaxel, dem Sulfonat oder Salz und Cisplatin einmal alle 2 - 4 Wochen behandelt wird, wobei die Dosierung von Paclitaxel von 160 mg/m² bis 190 mg/m² reicht, die Dosierung von dem Sulfonat oder Salz von 14 g/m² bis 22 g/m² reicht und die Dosierung von Cisplatin von 60 mg/m² bis 100 mg/m² reicht, wobei die Verabreichung von Paclitaxel, dem Sulfonat oder Salz und Cisplatin einmal alle 2 - 4 Wochen mindestens einmal wiederholt wird.

8. 2,2'-Dithio-bis-ethansulfonat oder ein pharmazeutisch akzeptables Salz davon zur Verwendung nach Anspruch 1, wobei der Patient an Lungenkrebs leidet und durch die Verabreichung von Paclitaxel, dem Sulfonat oder Salz und Cisplatin einmal alle 3 Wochen behandelt wird, wobei die Dosierung von Paclitaxel etwa 175 mg/m² ist, die Dosierung von dem Sulfonat oder Salz etwa 18,4 g/m² ist und die Dosierung von Cisplatin von 75 mg/m² bis 85 mg/m² reicht, wobei die Verabreichung von Paclitaxel, dem Sulfonat oder Salz und Cisplatin einmal alle 3 Wochen mindestens für 6 Zyklen wiederholt wird.

9. 2,2'-Dithio-bis-ethansulfonat oder ein pharmazeutisch akzeptables Salz davon zur Verwendung nach Anspruch 1, wobei ein Taxan-Medikament und Cisplatin und das Sulfonat oder Salz an einen Patienten mit Lungenkrebs oder einem Adenokarzinom verabreicht werden, wobei; (a) die Dosierungshäufigkeiten von dem Taxan-Medikament und dem Cisplatin von 10 - 20 mg/m²/Tag reichen und eine Dosierungshäufigkeit von dem Sulfonat oder Salz von 4,1 - 41,0 g/m² pro Tag reicht; (b) die Konzentration von dem Taxan-Medikament und dem Cisplatin und/oder dem Sulfonat oder Salz mindestens 0,01 mg/ml ist; (c) die Infusionszeit von dem Taxan-Medikament und dem Cisplatin und/oder dem Sulfonat oder Salz von 5 Minuten bis 24 Stunden reicht und nach Bedarf und Tolerierung an einem gegebenen Patienten wiederholt werden kann; und (d) der Verabreichungszeitplan von dem Taxan-Medikament und dem Cisplatin und/oder Sulfonat oder Salz alle 2 - 8 Wochen ist.

## Revendications

1. 2,2'-dithio-bis-éthane sulfonate ou sel pharmaceutiquement acceptable de celui-ci, pour une utilisation dans l'atténuation ou la prévention d'une anémie induite par chimiothérapie chez un patient, dans lequel le patient souffre d'un cancer du poumon ou d'un adénocarcinome et est traité avec un médicament taxane et/ou du cisplatine.

2. 2,2'-dithio-bis-éthane sulfonate ou sel pharmaceutiquement acceptable de celui-ci, pour une utilisation selon la revendication 1, dans lequel ledit cancer du poumon est un carcinome pulmonaire non à petites cellules.

3. 2,2'-dithio-bis-éthane sulfonate ou sel pharmaceutiquement acceptable de celui-ci, pour une utilisation selon la revendication 1 ou 2, dans lequel ladite anémie est atténuée ou prévenue chez ledit patient par :
a) maintien, protection ou stimulation de l'érythropolèse dans la moelle osseuse ;
b) augmentation de la synthèse ou de la demi-vie de l'érythropoïétine ;
c) maintien de la régulation physiologique normale de la biosynthèse, de la sécrétion, et rétrocontrôle de l'érythropoïèse ; et/ou
d) diminution du renouvellement des érythrocytes en circulation.

4. 2,2'-dithio-bis-éthane sulfonate ou sel pharmaceutiquement acceptable de celui-ci, pour une utilisation selon l'une quelconque des revendications 1 à 3, dans lequel ledit sel est le 2,2'-dithio-bis-éthane sulfonate disodique.

5. 2,2'-dithio-bis-éthane sulfonate ou sel pharmaceutiquement acceptable de celui-ci, pour une utilisation selon l'une quelconque des revendications 1 à 4, dans lequel ledit patient est traité avec un médicament taxane choisi dans le groupe consistant en : le docétaxel, le paclitaxel, les formes polyglutamylées du paclitaxel et le paclitaxel liposomal.

6. 2,2'-dithio-bis-éthane sulfonate ou sel pharmaceutiquement acceptable de celui-ci, pour une utilisation selon l'une quelconque des revendications 1 à 5, dans lequel ledit patient est traité avec du cisplatine et du paclitaxel.

7. 2,2'-dithio-bis-éthane sulfonate ou sel pharmaceutiquement acceptable de celui-ci, pour une utilisation selon la revendication 1, dans lequel ledit patient souffre d'un cancer du poumon et doit être traité par l'administration de paclitaxel, de sulfonate ou sel, et de cisplatine une fois toutes les 2 à 4 semaines, dans lequel la dose de paclitaxel doit varier de 160 mg/m² à 190 mg/m², la dose de sulfonate ou sel doit varier de 14 g/m² à 22 g/m², et la dose de cisplatine doit varier de 60 mg/m² à 100 mg/m², dans lequel ladite administration de paclitaxel, de sulfonate ou sel et du cisplatine une fois toutes les 2 à 4 semaines doit être répétée au moins une fois.

8. 2,2'-dithio-bis-éthane sulfonate ou sel pharmaceutiquement acceptable de celui-ci, pour une utilisation selon la revendication 1, dans lequel ledit patient souffre d'un cancer du poumon et doit être traité par l'administration de paclitaxel, de sulfonate ou sel, et de cisplatine une fois toutes les 3 semaines, dans lequel la dose de paclitaxel doit être approximativement de 175 mg/m², la dose de sulfonate ou sel doit être approximativement de 18,4 mg/m², et la dose de cisplatine doit varier de 75 mg/m² à 85 mg/m², dans lequel ladite administration de paclitaxel, de sulfonate ou sel et de cisplatine une fois toutes les 3 semaines doit être répétée pendant 6 cycles.

9. 2,2'-dithio-bis-éthane sulfonate ou sel pharmaceutiquement acceptable de celui-ci, pour une utilisation selon la revendication 1, dans lequel un médicament taxane et du cisplatine et le sulfonate ou sel doivent être administrés à un patient atteint d'un cancer du poumon ou d'un adénocarcinome, dans lequel : (a) l'intensité de la dose du médicament taxane et du cisplatine doit varier de 10 à 20 mg/m²/jour et l'intensité de la dose du sulfonate ou sel doit varier de 4,1 à 41,0 g/m² par jour ; (b) la concentration du médicament taxane et du cisplatine et/ou du sulfonate ou sel est d'au moins 0,01 mg/mL ; (c) le temps d'infusion du médicament taxane et du cisplatine et/ou du sulfonate ou sel est de 5 minutes à 24 heures, et peut être répété si nécessaire et toléré chez un patient donné ; et (d) le programme d'administration du médicament taxane et du cisplatine et/ou du sulfonate ou sel est toutes les 2 à 8 semaines.
